(19) Europäisches Patentamt
European Patent Office
Office européen des brevets

(11) Publication number: 0 644 191 A1

(12) # EUROPEAN PATENT APPLICATION published in accordance with Art. 158(3) EPC

(21) Application number: 93910422.0

(22) Date of filing: 31.05.93

(86) International application number: PCT/JP93/00732

(87) International publication number: WO 93/24480 (09.12.93 93/29)

(51) Int. Cl.6: C07D 401/12, C07D 401/14, C07D 413/12, C07D 413/14, C07D 417/12, C07D 417/14, C07D 471/04, C07D 498/04, C07D 513/04, A61K 31/44, A61K 31/535

(30) Priority: 01.06.92 JP 167017/92

(43) Date of publication of application: 22.03.95 Bulletin 95/12

(84) Designated Contracting States: AT BE CH DE DK ES FR GB GR IE IT LI LU MC NL PT SE

(71) Applicant: **YOSHITOMI PHARMACEUTICAL INDUSTRIES, LTD.**
**6-9, Hiranomachi 2-chome**
**Chuo-ku**
**Osaka-shi**
**Osaka 541 (JP)**

(72) Inventor: **KAWAKITA, Takeshi, Yoshitomi Pharm. Ind. Ltd.**
**Discovery Research Lab. II,**
**955, Oaza-Koiwai**
**Yoshitomimachi, Chikujo-gun,**
**Fukuoka 871 (JP)**
Inventor: **YUTOKU, Yuko, Yoshitomi Pharm. Ind. Ltd.**
**Discovery Research Lab. II,**
**955, Oaza-Koiwai**
**Yoshitomimachi, Chikujo-gun,**
**Fukuoka 871 (JP)**
Inventor: **HAGA, Keiichiro, Yoshitomi Pharm. Ind. Ltd.**
**Discovery Research Lab. II,**
**955, Oaza-Koiwai**
**Yoshitomimachi, Chikujo-gun,**
**Fukuoka 871 (JP)**
Inventor: **IKEDA, Yoshifumi, Yoshitomi Pharm. Ind. Ltd.**
**Discovery Research Lab. II,**
**955, Oaza-Koiwai**
**Yoshitomimachi, Chikujo-gun,**
**Fukuoka 871 (JP)**

(74) Representative: **von Kreisler, Alek, Dipl.-Chem. et al**
**Patentanwälte**
**von Kreisler-Selting-Werner**
**Bahnhofsvorplatz 1 (Deichmannhaus)**
**D-50667 Köln (DE)**

(54) **PYRIDINE COMPOUND AND MEDICINAL USE THEREOF.**

(57) A pyridine compound represented by general formula (I) or a pharmaceutical acceptable salt thereof, having the antibacterial effect against *Helicobacter pylori*, antiulcer effect, the effects of protecting gastrointestinal cells and inhibiting the recrudescence and recurrence of ulcer, and so forth, thus being useful as a medicine, wherein $R^1$ represents hydrogen, halogen, alkyl, alkoxy, etc.; $R^2$ and $R^3$ represent each hydrogen, halogen or alkyl; -P=Q- represents -CH=CH-, -N=CH- or -CH=N-; A represents oxygen, sulfur or $NR^4$ (wherein $R^4$ represents hydrogen, alkyl, etc.); n represents 0, 1 or 2; B represents oxygen, S(O)p (wherein p represents 0, 1 or 2) or $NR^5$ (wherein $R^5$ represents hydrogen or alkyl); D represents a single bond, alkylene, etc.; and E represents alkoxyalkyl or $-NR^6R^7$.

EP 0 644 191 A1

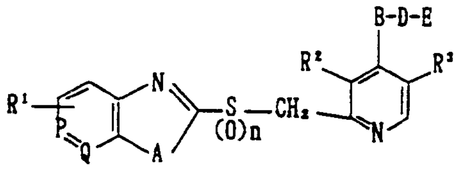

(I)

TECHNICAL FIELD

The present invention relates to a novel pyridine compound having antiulcer activity, gastrointestinal cytoprotective activity, ulcer recurrence or relapse-preventive activity, gastric acid secretion-suppressive activity and/or antibacterial activity against *Helicobacter pylori*, and a pharmaceutically acceptable salt thereof.

BACKGROUND ART

Of the compounds recently developed as antiulcer preparations, 5-methoxy-2-[((4-methoxy-3,5-dimethyl-2-pyridyl)methyl)sulfinyl]-1H-benzimidazole (generally called omeprazole) as disclosed in Japanese Patent Publication No. 34956/1985 has been acknowledged of its clinical utility.

Thereafter, a number of compounds have been reported and Japanese Patent Unexamined Publication Nos. 181277/1984, 6270/1989, 79177/1989 and International Publication No. WO89/00566 disclose compounds having gastric acid secretion-suppressive activity and useful as antiulcer drugs. In addition, Japanese Patent Unexamined Publication Nos. 48680/1991, 52887/1991, 173817/1991 and International Publication No. WO92/12976 disclose a series of compounds having antibacterial activity against *Helicobacter pylori* (old name: *Campylobacter pylori*).

While the treatment of patients with peptic ulcer has been making rapid progress ever since a gastric acid secretion suppressor was developed, problems of recurrence and relapse of ulcer have also arisen. For example, the percent relapse in 12 months after the treatment with an $H_2$ antagonist is reportedly very high and is 70-90%.

A high percentage in the detection of *Helicobacter pylori* in the patients with gastritis or peptic ulcer suggests involvement of *Helicobacter pylori* in gastritis and peptic ulcer [D.Y. Graham, Gastroenterology, *96*, p. 615 (1989)]. Also, reports with regard to decreased recurrence and relapse ratio of ulcer by the eradication of *Helicobacter pylori* are increasing in number [The Lancet, *336*, p. 755 (1990), ibid, *337*, p. 1614 (1991)].

For the eradication of *Helicobacter pylori*, antibiotics and bismuth preparations have been used. Antibiotics, however, exert influences on other bacteria as well and a long-term use of them is not desirable. On the other hand, bismuth preparations have low potency and cause side effects such as vomiting, diarrhea and central side effects.

Accordingly, the treatment of bacterial infections with *Helicobacter pylori* requires selective and highly antibacterial drugs. For the prevention of recurrence and relapse of ulcer, a novel pharmaceutical preparation having, besides these specific antibacterial and bacteriocidal activity, antiulcer activity and gastric acid secretion-suppressive activity is desired to be developed.

With the aim of solving the above-mentioned problems, the present inventors have conducted various studies and found a compound having not only selective and superior antibacterial activity against *Helicobacter pylori*, but also antiulcer activity, gastrointestinal cytoprotective activity and gastric acid secretion-suppressive activity, which resulted in the completion of the invention.

DISCLOSURE OF THE INVENTION

The present invention is as follows.

1. A pyridine compound of the formula (I)

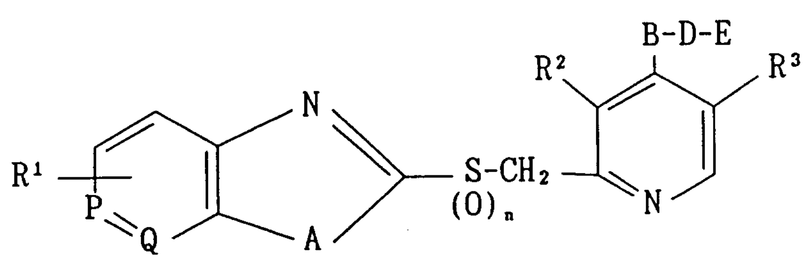

(I)

wherein

$R^1$ is a hydrogen, a halogen, an alkyl, an alkoxy, a hydroxyl, an alkoxycarbonyl, a carboxyl, a haloalkyl, a nitro, an amino, a mono- or dialkylamino, an alkoxycarbonylalkylamino or a carboxyal-

kylamino,

$R^2$ and $R^3$ are the same or different and each is a hydrogen, a halogen or an alkyl,

-P = Q- is -CH = CH-, -N = CH- or -CH = N-,

A is an oxygen atom, a sulfur atom or $N(R^4)$ wherein $R^4$ is hydrogen, alkyl, alkoxycarbonyl, hydroxyalkyl, alkoxyalkyl, acyloxyalkyl, alkoxycarbonylalkyl, carboxyalkyl, carbamoyl, carbamoylalkyl, mono- or dialkylcarbamoyl, mono- or dialkylcarbamoylalkyl, thiocarbamoyl, or mono- or dialkylthiocarbamoyl,

n is 0, 1 or 2,

B is an oxygen atom, S(O)p wherein p is 0, 1 or 2 or $N(R^5)$ wherein $R^5$ is hydrogen or alkyl,

D is a single bond, an alkylene, an alkylene having substituent or an alkylene having oxo, and

E is an alkoxyalkyl, a group of the formula (a)

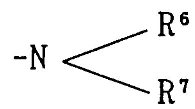

(a)

wherein $R^6$ and $R^7$ are the same or different and each is hydrogen, alkyl, cycloalkyl, acyl, alkoxycarbonyl, carbamoyl, mono- or dialkylcarbamoyl, optionally substituted, phenylcarbamoyl, thiocarbamoyl, mono- or dialkylthiocarbamoyl, optionally substituted phenylthiocarbamoyl, hydroxyalkyl, alkoxycarbonylalkyl, optionally substituted phenylalkylcarbamoyl, optionally substituted phenylalkylthiocarbamoyl, carboxyalkyl, optionally substituted phenyl, optionally substituted phenylalkyl or optionally substituted heteroarylalkyl, or $R^6$ and $R^7$ may form, together with the adjoining nitrogen atom, an optionally condensed and optionally substituted heterocyclic ring, or a group of the formula (b)

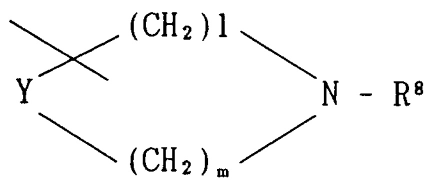

(b)

wherein $R^8$ is hydrogen, alkyl, acyl, carboxyalkyl or optionally substituted phenylalkyl, Y is methylene, oxygen atom or sulfur atom and l and m are the same or different and each is 0 or an integer of 1-3;

provided that when -P = Q- is -CH = CH- and A is $N(R^4)$, either one of the following applies:

(1) when $R^4$ is hydrogen, B is S(O)p wherein p is 0, 1 or 2 or $N(R^5)$ wherein $R^5$ is hydrogen or alkyl;

(2) when $R^4$ is carbamoyl or mono- or dialkylcarbamoyl, B is oxygen atom, D is alkylene and E is alkoxy, D is alkylene having 3 to 10 carbon atoms;

(3) when $R^4$ is carbamoyl or mono- or dialkylcarbamoyl, B is oxygen atom, D is alkylene and E is a group of the formula (a), D is alkylene having 5 to 10 carbon atoms or in the formula (a), $R^6$ and $R^7$ are the same or different and each is hydrogen, alkyl having 3 to 20 carbon atoms, cycloalkyl, acyl, optionally substituted phenyl, optionally substituted phenylalkyl or optionally substituted heteroarylalkyl, or $R^6$ and $R^7$ may form, together with the adjoining nitrogen atom, an optionally condensed and optionally substituted heterocyclic ring; and

(4) when $R^4$ is alkyl having 1 to 6 carbon atoms or alkoxycarbonyl having 2 to 7 carbon atoms, D is alkylene and E is alkoxy, B is S(O)p wherein p is 0, 1 or 2 or $N(R^5)$ wherein $R^5$ is hydrogen or alkyl;

and a pharmaceutically acceptable salt thereof.

2. The pyridine compound of the above 1. having the formula (I) wherein B is S(O)p wherein p is 0, 1 or 2 and other symbols are as defined above in 1. and a pharmaceutically acceptable salt thereof.

3. The pyridine compound of the above 1., which is selected from the group of
2-(3-methyl-4-(2-morpholinoethylthio)-2-pyridyl)methylthio-1H-benzimidazole,
2-(3-methyl-4(2-(1,2,3,4-tetrahydroisoquinolin-2-yl)ethylthio)-2-pyridyl)methylthio-1H-benzimidazole,
2-(4-(2-(N-benzyl-N-methylamino)ethylthio)-3-methyl-2-pyridyl)methylthio-1H-benzimidazole,
2-(4-(1-benzyl-4-piperidyl)thio-3-methyl-2-pyridyl)methylthio-1H-benzimidazole,
2-((3-methyl-4-(2-(1,2,3,4-tetrahydroisoquinolin-2-yl)ethylthio)-2-pyridyl)methylthio)imidazo[5,4-b]pyridine,
ethyl 2-(3-methyl-4-(2-morpholinoethylthio)-2-pyridyl)methylthio-1H-benzimidazole-1-carboxylate,

methyl 2-(3-methyl-4-(2-(1,2,3,4-tetrahydroisoquinolin-2-yl)ethylthio)-2-pyridyl)methylthio-1H-benzimidazole-1-carboxylate, 4-(2-((2-(1H-benzimidazol-2-yl)thiomethyl-3-methyl-4-pyridyl)thio)ethyl)-morpholine N-oxide,
2-(3-methyl-4-(2-acetylaminoethylthio)-2-pyridyl)methylthio-1H-benzimidazole,
2-(3-methyl-4-(2-benzylaminoethylthio)-2-pyridyl)methylthio-1H-benzimidazole,
2-(3-methyl-4-(2-(2-hydroxyethylamino)ethylthio)-2-pyridyl)methylthio-1H-benzimidazole, 2-(3-methyl-4-(2-(N,N-di(2-hydroxyethyl)amino)ethylthio)-2-pyridyl)methylthio-1H-benzimidazole,
2-(3-methyl-4-(2-(2-carboxyethylamino)ethylthio)-2-pyridyl)methylthio-1H-benzimidazole and
2-(3-methyl-4-(2-morpholino-2-carboxyethylthio)-2-pyridyl)methylthio-1H-benzimidazole, and a pharmaceutically acceptable salt thereof.

4. A pharmaceutical composition comprising the compound of the above 1. and pharmaceutical additives.

5. A preparation for preventing or treating various diseases caused by the bacteria belonging to the genus ***Helicobacter,*** which comprises the compound of the above 1. as an active ingredient.

6. A preparation for preventing or treating gastrointestinal diseases, which comprises the compound of the above 1. as an active ingredient.

Specific examples of the respective groups in the formula (I) are as follows.

With regard to $R^1$, halogen is chlorine, fluorine, bromine or iodine, alkyl is that having 1 to 20 carbon atoms, such as methyl, ethyl, propyl, isopropyl, butyl, isobutyl, tert-butyl, pentyl, hexyl, octyl, decyl, dodecyl, octadecyl and icosyl, alkoxy is that having 1 to 20 carbon atoms, such as methoxy, ethoxy, propoxy, isopropoxy, butoxy, isobutoxy, tert-butoxy, pentyloxy, hexyloxy, octyloxy, decyloxy, dodecyloxy, octadecyloxy and icosyloxy, alkoxycarbonyl is that having 2 to 21 carbon atoms, such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl, octyloxycarbonyl, decyloxycarbonyl, dodecyloxycarbonyl, octadecyloxycarbonyl and icosyloxycarbonyl, haloalkyl is that having 1 to 4 carbon atoms, such as trifluoromethyl, 2,2,2-trifluoroethyl, 2,3,3-trifluoropropyl, 1,1,2,2-tetrafluoroethyl and 2,2,3,3-tetrafluoropropyl, mono- or dialkylamino is methyl amino, dimethylamino, ethylamino, diethylamino, propylamino, dipropylamino, butylamino or dibutylamino, particularly mono- or di- $C_1$-$C_4$ alkylamino,alkoxycarbonylalkylamino is methoxycarbonylmethylamino, ethoxycarbonylmethylamino, propoxycarbonylmethylamino, isopropoxycarbonyl methyl amino, butoxycarbonylmethylamino, isobutoxycarbonylmethylamino, tert-butoxycarbonylmethylamino, 2-methoxycarbonylethylamino or 2-ethoxycarbonylethylamino, particularly $C_1$-$C_4$ alkoxycarbonyl $C_1$-$C_4$ alkylamino, and carboxyalkylamino is carboxymethylamino, 2-carboxyethylamino, 3-carboxypropylamino or 4-carboxybutylamino, particularly carboxy $C_1$-$C_4$ alkylamino.

With regard to $R^2$ and $R^3$, halogen is chlorine, fluorine, bromine or iodine, alkyl is that having 1 to 4 carbon atoms, such as methyl, ethyl, propyl, isopropyl, butyl, isobutyl and tert-butyl, $R^2$ is preferably methyl and $R^3$ is preferably hydrogen or methyl.

With regard to $R^4$, alkyl is that having 1 to 20 carbon atoms, such as methyl, ethyl, propyl, isopropyl, butyl, isobutyl, tert-butyl, pentyl, hexyl, octyl, decyl, dodecyl, octadecyl and icosyl, alkoxycarbonyl is that having 2 to 21 carbon atoms, such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl, octyloxycarbonyl, decyloxycarbonyl, dodecyloxycarbonyl, octadecyloxycarbonyl and icosyloxycarbonyl, hydroxyalkyl is that having 1 to 4 carbon atoms, such as hydroxymethyl, 1-hydroxyethyl, 2-hydroxyethyl, 3-hydroxypropyl, 2,3-dihydroxypropyl and 4-hydroxybutyl, and alkoxyalkyl is methoxymethyl, ethoxymethyl, propoxymethyl, isopropoxymethyl, butoxymethyl, isobutoxymethyl, tert-butoxymethyl, 2-methoxyethyl, 2-ethoxyethyl, 3-methoxypropyl or 4-methoxybutyl, particularly $C_1$-$C_4$ alkoxy $C_1$-$C_4$ alkyl. Acyloxyalkyl is acetyloxymethyl, propionyloxymethyl, 2-acetyloxyethyl, 3-acetyloxypropyl, 4-acetyloxybutyl, benzoyloxymethyl, 2-benzoyloxyethyl, or benzoyloxymethyl or 2-benzoyloxyethyl substituted by 1 to 3 substituents selected from halogen, alkyl, alkoxy, haloalkyl, hydroxyl, nitro and amino at phenyl, particularly $C_1$-$C_7$ acyloxy $C_1$-$C_4$ alkyl. Alkoxycarbonylalkyl is methoxycarbonylmethyl, ethoxycarbonylpropyl, propoxycarbonylethyl, isopropoxycarbonylethyl, butoxycarbonylmethyl, isobutoxycarbonylethyl, tert-butoxycarbonylmethyl, pentyloxycarbonylmethyl, hexyloxycarbonylmethyl, octyloxycarbonylmethyl, decyloxycarbonylethyl, dodecyloxycarbonylmethyl, octadecyloxycarbonylmethyl or icosyloxycarbonylmethyl, particularly $C_1$-$C_{20}$ alkoxycarbonyl $C_1$-$C_4$ alkyl. Carboxyalkyl is that having 1 to 5 carbon atoms, such as carboxymethyl, 2-carboxyethyl, 3-carboxypropyl and 4-carboxybutyl.

Carbamoylalkyl is carbamoylmethyl, 2-carbamoylethyl, 3-carbamoylpropyl or 4-carbamoylbutyl, particularly carbamoyl $C_1$-$C_4$ alkyl. Mono- or dialkylcarbamoyl is carbamoyl substituted by alkyl at its nitrogen atom, such as N-methylcarbamoyl, N,N-dimethylcarbamoyl, N-ethylcarbamoyl, N,N-diethylcarbamoyl, N-propylcarbamoyl, N,N-dipropylcarbamoyl, N-isopropylcarbamoyl and N-butylcarbamoyl, particularly mono-

or di- $C_1$-$C_4$ alkylcarbamoyl. Mono- or dialkylcarbamoylalkyl is carbamoyl substituted by alkyl at its nitrogen atom, such as N-methylcarbamoylmethyl, N,N-dimethylcarbamoylmethyl, N-ethylcarbamoylmethyl, N,N-diethylcarbamoylmethyl, N-propylcarbamoylmethyl, N,N-dipropylcarbamoylmethyl, N-isopropylcarbamoylmethyl and N-butylcarbamoylmethyl, particularly mono- or di- $C_1$-$C_4$ alkylcarbamoyl $C_1$-$C_4$ alkyl. Mono- or dialkylthiocarbamoyl is thiocarbamoyl substituted by alkyl at its nitrogen atom, such as N-methylthiocarbamoyl, N,N-dimethylthiocarbamoyl, N-ethylthiocarbamoyl, N,N-diethylthiocarbamoyl, N-propylthiocarbamoyl, N,N-dipropylthiocarbamoyl, N-isopropylthiocarbamoyl and N-butylthiocarbamoyl, particularly mono- or di- $C_1$-$C_4$ alkylthiocarbamoyl.

Alkyl at $R^5$ is that having 1 to 4 carbon atoms, such as methyl, ethyl, propyl, isopropyl, butyl, isobutyl and tert-butyl.

Alkylene at D is that having 1 to 10 carbon atoms, such as methylene, ethylene, propylene, trimethylene, tetramethylene, pentamethylene, hexamethylene, heptamethylene, octamethylene, nonamethylene and decamethylene, which may have branched chain. Alkylene having substituent is that having substituent such as hydroxy, hydroxyalkyl (e.g. hydroxymethyl, 2-hydroxyethyl, 3-hydroxypropyl, particularly hydroxy $C_1$-$C_4$ alkyl), alkoxycarbonyl (e.g. methoxycarbonyl, ethoxycarbonyl, propoxycarbonyl, butoxycarbonyl, particularly $C_1$-$C_4$ alkoxycarbonyl), carboxyl, carbamoyl, or mono- or dialkylcarbamoyl (e.g. methylcarbamoyl, dimethylcarbamoyl, ethylcarbamoyl, diethylcarbamoyl, propylcarbamoyl, dipropylcarbamoyl, particularly mono- or di- $C_1$-$C_4$ alkylcarbamoyl), at an optional position of alkylene, which is exemplified by 2-hydroxytrimethylene, 1-hydroxymethylethylene, 2-carboxyethylene, 2-ethoxycarbonylethylene, 4-ethoxycarbonyltetramethylene, 2-carbamoylethylene, 2-N-methylcarbamoylethylene, 2-N-ethylcarbamoylethylene and 2-N,N-dimethylcarbamoylethylene. Alkylene having oxo is, for example, $-CH_2CO-$, $-CH_2CH_2CO-$, $-CH_2CH_2CH_2CO-$ or $-CH_2CH_2CH_2CH_2CO-$.

Alkoxyalkyl at E is methoxymethyl, ethoxymethyl, propoxymethyl, isopropoxymethyl, butoxymethyl, isobutoxymethyl, tert-butoxymethyl, pentyloxymethyl, hexyloxymethyl, octyloxymethyl, decyloxymethyl, dodecyloxymethyl, octadecyloxymethyl, icosyloxymethyl, 2-methoxyethyl, 3-methoxypropyl, 3-propoxypropyl, 4-methoxybutyl or 4-isopropoxybutyl, particularly $C_1$-$C_{20}$ alkoxy $C_1$-$C_4$ alkyl.

With regard to $R^6$ and $R^7$, alkyl is that having 1 to 20 carbon atoms, such as methyl, ethyl, propyl, isopropyl, butyl, isobutyl, tert-butyl, pentyl, hexyl, octyl, decyl, dodecyl, octadecyl and icosyl and cycloalkyl is that having 3 to 7 carbon atoms, such as cyclopropyl, cyclobutyl, cyclopentyl, cyclohexyl and cycloheptyl. Acyl is alkanoyl having 1 to 5 carbon atoms, such as acetyl, propionyl, isopropionyl, butyryl, pivaloyl and valeryl, or benzoyl. Alkoxycarbonyl is alkoxycarbonyl having 2 to 21 carbon atoms, such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl, octyloxycarbonyl, decyloxycarbonyl, dodecyloxycarbonyl, octadecyloxycarbonyl and icosyloxycarbonyl, mono- or dialkylcarbamoyl is carbamoyl substituted by alkyl at its nitrogen atom, which is exemplified by N-methylcarbamoyl, N,N-dimethylcarbamoyl, N-ethylcarbamoyl, N,N-diethylcarbamoyl, N-propylcarbamoyl, N,N-dipropylcarbamoyl, N-isopropylcarbamoyl and N-butylcarbamoyl, particularly mono- or di- $C_1$-$C_4$ alkylcarbamoyl. Mono- or dialkylthiocarbamoyl is thiocarbamoyl substituted by alkyl at its nitrogen atom, which is exemplified by N-methylthiocarbamoyl, N,N-dimethylthiocarbamoyl, N-ethylthiocarbamoyl, N,N-diethylthiocarbamoyl, N-propylthiocarbamoyl, N,N-dipropylthiocarbamoyl, N-isopropylthiocarbamoyl and N-butylthiocarbamoyl, particularly mono- or di- $C_1$-$C_4$ alkylthiocarbamoyl. Hydroxyalkyl is that having 1 to 5 carbon atoms, such as hydroxymethyl, 1-hydroxyethyl, 2-hydroxyethyl, 2-hydroxypropyl, 3-hydroxypropyl, 2,3-dihydroxypropyl, 4-hydroxybutyl and 5-hydroxypentyl. Alkoxycarbonylalkyl is methoxycarbonylmethyl, ethoxycarbonylmethyl, propoxycarbonylethyl, isopropoxycarbonylethyl, butoxycarbonylmethyl, isobutoxycarbonylethyl, tert-butoxycarbonylmethyl, pentyloxycarbonylmethyl, hexyloxycarbonylmethyl, octyloxycarbonylmethyl, decyloxycarbonylethyl, dodecyloxycarbonylmethyl, octadecyloxycarbonylmethyl or icosyloxycarbonylmethyl, particularly $C_1$-$C_{20}$ alkoxycarbonyl $C_1$-$C_4$ alkyl. Carboxyalkyl is that having 2 to 5 carbon atoms, such as carboxymethyl, 2-carboxyethyl, 3-carboxypropyl and 4-carboxybutyl. Phenylalkylcarbamoyl is benzylcarbamoyl, 2-phenylethylcarbamoyl or 3-phenylpropylcarbamoyl, particularly phenyl $C_1$-$C_4$ alkylcarbamoyl. Phenylalkylthiocarbamoyl is benzylthiocarbamoyl, 2-phenylethylthiocarbamoyl or 3-phenylpropylthiocarbamoyl, particularly phenyl $C_1$-$C_4$ alkylthiocarbamoyl. Phenylalkyl is phenyl-substituted alkyl having 1 to 8 carbon atoms, such as benzyl, 1-phenylethyl, 2-phenylethyl, 3-phenylpropyl, 4-phenylbutyl, 6-phenylhexyl and 8-phenyloctyl. Heteroarylalkyl is alkyl having 1 to 4 carbon atoms which is substituted by heteroaryl such as furyl, thienyl, pyridyl and pyrimidinyl and is exemplified by 2-thenyl, 3-thenyl, furfuryl, 3-furylmethyl, 2-, 3- or 4-pyridylmethyl and 2-pyrimidinylmethyl. The substituents for the optionally substituted phenyl, phenylcarbamoyl, phenylthiocarbamoyl, phenylalkylcarbamoyl, phenylalkylthiocarbamoyl, phenylalkyl and heteroarylalkyl are, for example, 1 to 3 groups selected from halogen (e.g. chlorine, fluorine, bromine, iodine), alkyl (e.g. $C_1$-$C_4$ alkyl such as methyl, ethyl, propyl, isopropyl, butyl, isobutyl and tert-butyl), alkoxy

(e.g. $C_1$-$C_4$ alkoxy such as methoxy, ethoxy, propoxy, isopropoxy, butoxy, isobutoxy and tert-butoxy), haloalkyl (e.g. halo $C_1$-$C_4$ alkyl such as trifluoromethyl and 2,2,2-trifluoroethyl), hydroxy, nitro and amino.

Optionally condensed heterocyclic ring formed by $R^6$ and $R^7$ together with the adjoining nitrogen atom is, for example, 1-pyrrolidinyl, piperidino, 1-piperazinyl, 4-alkyl-1-piperazinyl, 4-phenyl-1-piperazinyl, 4-substituted phenyl-1-piperazinyl, 4-aralkyl-1-piperazinyl, 4-substituted aralkyl-1-piperazinyl, 4-acyl-1-piperazinyl, 4-alkyl-1-homopiperazinyl, 4-acyl-1-homopiperazinyl, morpholino, thiomorpholino, 2-oxo-1-pyrrolidinyl, 2,4-dioxo-1-pyrrolidinyl, 2-oxo-1-piperidinyl, isoindolin-2-yl, 1,2,3,4-tetrahydroquinolin-1-yl or 1,2,3,4-tetrahydroisoquinolin-2-yl. The morpholino and thiomorpholino may be substituted by carboxyl, hydroxyalkyl (e.g. hydroxymethyl, 2-hydroxyethyl, 3-hydroxypropyl), aminoalkyl (e.g. aminomethyl, 2-aminoethyl), mono- or dialkylaminoalkyl (e.g. methylaminomethyl, dimethylaminomethyl, 2-dimethylaminoethyl), alkoxycarbonyl (e.g. methoxycarbonyl, ethoxycarbonyl, propoxycarbonyl, isopropoxycarbonyl, butoxycarbonyl), carboxyalkoxyalkyl (e.g. carboxymethoxymethyl, 2-carboxyethoxymethyl, 3-carboxypropoxymethyl), alkoxycarbonylalkoxyalkyl (e.g. methoxycarbonylmethoxymethyl, ethoxycarbonylmethoxymethyl, 2-ethoxycarbonylethoxymethyl, 3-ethoxycarbonylpropoxymethyl), carboxyalkylaminoalkyl (e.g. carboxymethylaminomethyl, 2-carboxyethylaminomethyl, 3-carboxypropylaminomethyl), mono- or dihydroxyalkylamino (e.g. hydroxymethylamino, 2-hydroxyethylamino, 2,3-dihydroxypropylamino), or mono- or dialkoxyalkylamino (e.g. methoxymethylamino, ethoxymethylamino, 2-ethoxyethylamino, 2,3-diethoxypropylamino). Alkyl which substitutes the 4-position of piperazine is, for example, methyl, ethyl, propyl, isopropyl or butyl; acyl is, for example, acetyl, propionyl or butyryl; aralkyl is, for example, benzyl, 2-phenylethyl or 3-phenylpropyl; and the substituents for phenyl and aralkyl are exemplified by halogen, alkyl and alkoxy. Also, the isoindoline ring and the 1,2,3,4-tetrahydro(iso)quinoline ring may be substituted by 1 to 3 substituents optionally selected from halogen, alkyl, alkoxy, haloalkyl, hydroxyl, nitro, amino and oxo.

Examples of the heterocyclic ring which $R^6$ and $R^7$ form together with the adjoining nitrogen atom include the following.

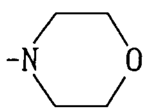 , 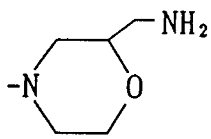 , 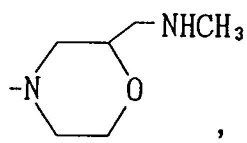 ,

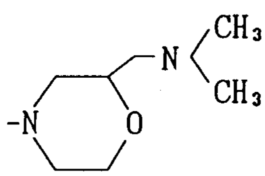 , 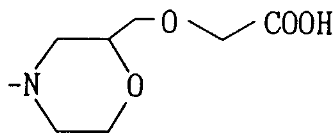 , 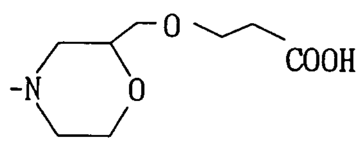 ,

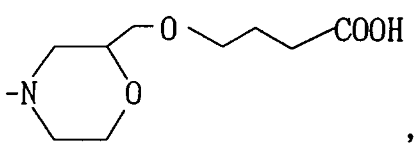 , 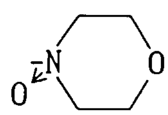 , 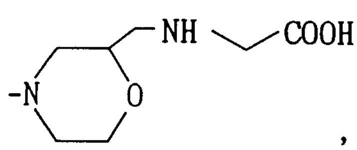 ,

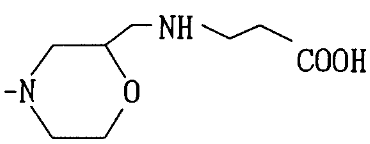 , 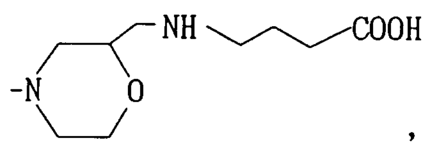 ,

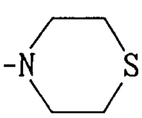 , 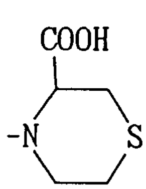 , 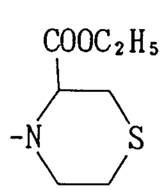 , 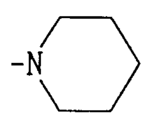 , 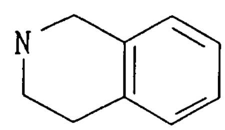 ,

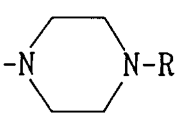 (R=H, $CH_3$, 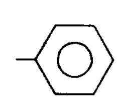, 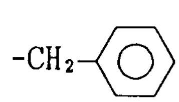 or $COCH_3$), 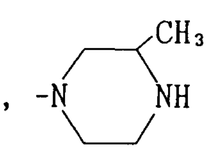

wherein alkyl, acyl, carboxyalkyl and optionally substituted phenyl alkyl at $R^8$ are as defined above.

The group of the formula (b) is exemplified by the following.

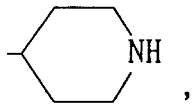 , 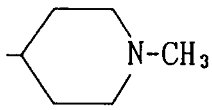 , 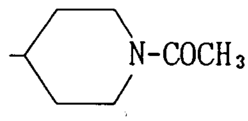 ,

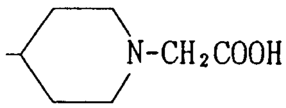 , 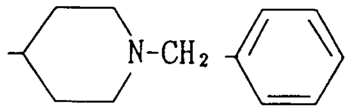 , 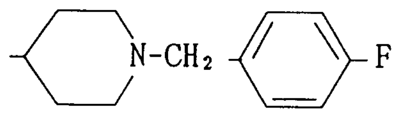 ,

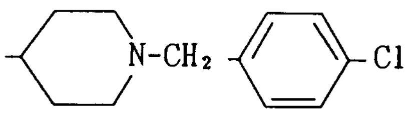 , 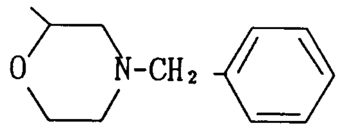 , 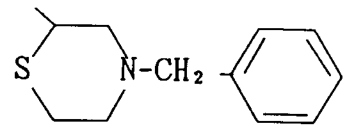

Preferable groups for E are as follows. $-NH_2$,

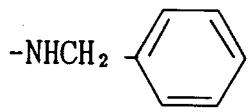 ,

$-NHCOCH_3$, $-NHCH_2CH_2OH$,

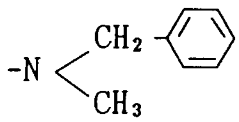 , 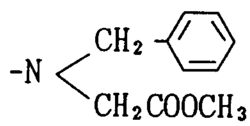 , 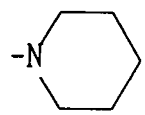 , 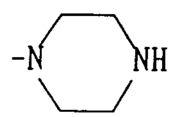 ,

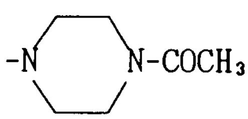 , 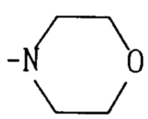 , 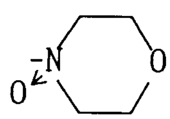 , 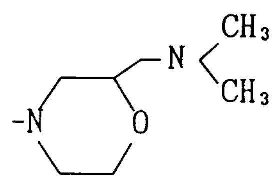 .

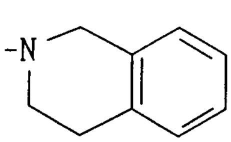 , 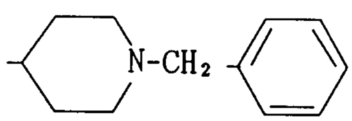 , 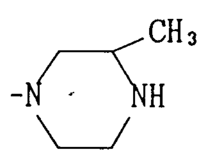

While the compound of the formula (I) of the present invention comprises various isomers, the present invention encompasses an isomer or a mixture of isomers.

The pharmaceutically acceptable salt of the compound of the formula (I) includes acid addition salts such as hydrochloride, hydrobromide, hydroiodide, sulfate, phosphate, acetate, citrate, maleate, fumarate, malonate, malate, tartrate, succinate, methanesulfonate, benzenesulfonate and a salt of the formula

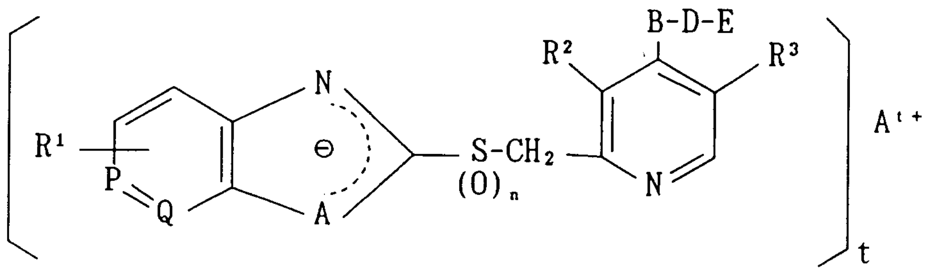

(I')

wherein t is 1, 2 or 4, $A^{t+}$ is $Li^+$, $Na^+$, $K^+$, $Mg^{2+}$, $Ca^{2+}$, $Ti^{4+}$ or $N^+(R)_4$ wherein R is alkyl having 1 to 4 carbon atoms and other symbols are as defined above. In the compounds of the formula (I'), sodium salt, potassium salt and magnesium salt are particularly preferable. The compound of the present invention may exist as hydrates (e.g. semi-hydrate, monohydrate, sesqui-hydrate) or solvates, which are also encompassed in the present invention.

The compounds of the formula (I) can be produced by the following methods.

Method 1: A compound of the formula (II)

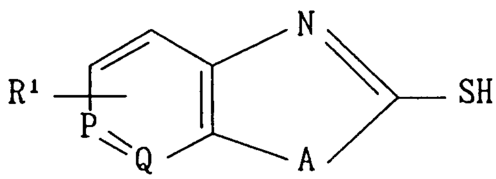

(II)

wherein each symbol is as defined above, is reacted with a compound of the formula (III)

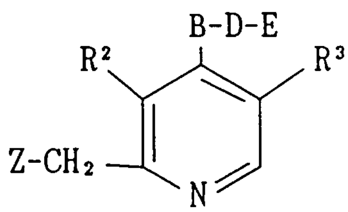

(III)

wherein Z is a reactive atom or group such as halogen or sulfonyloxy group (e.g. methanesulfonyloxy, benzenesulfonyloxy, p-toluenesulfonyloxy) and other symbols are as defined above, or an acid addition salt thereof to give a compound of the formula (I-1)

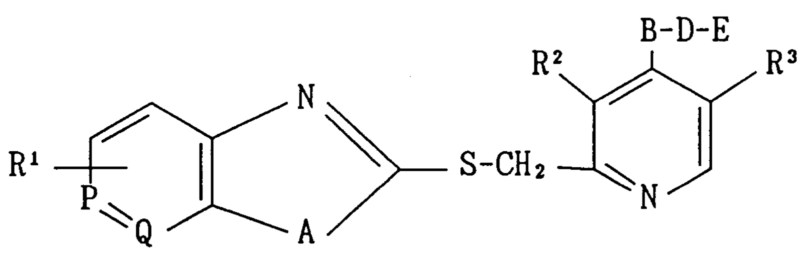

(I-1)

wherein each symbol is as defined above. A compound of the formula (I) wherein n is 1 or 2 can be obtained by subjecting a compound of the formula (I-1) wherein n is 0 to oxidation.

The reaction between a Compound (II) and a Compound (III) generally proceeds in a solvent inert to the reaction, such as water, methanol, ethanol, dimethylformamide and a mixed solvent thereof, preferably aqueous ethanol, in the presence of a base such as sodium hydroxide, potassium hydroxide, sodium hydride, potassium hydride, sodium methoxide, sodium carbonate, potassium carbonate, metallic sodium, triethylamine and pyridine at a temperature of from about 0 °C to the boiling point of the solvent used, preferably from 20 °C to 80 °C for about 10 minutes to 24 hours, preferably 30 minutes to 7 hours.

Examples of the oxidizing agent to be used for the oxidation include meta-chloroperbenzoic acid, peracetic acid, trifluoroperacetic acid, permaleic acid, potassium hyperoxide, sodium bromite, sodium hypobromite and hydrogen peroxide. The reaction is generally carried out in a solvent inert to the reaction such as water, dichloromethane, chloroform, tetrahydrofuran, dioxane, dimethylformamide and a mixed solvent thereof in the presence of an organic acid such as formic acid, acetic acid, propionic acid, butyric acid, maleic acid, fumaric acid, malonic acid, succinic acid, benzoic acid, metachlorobenzoic acid, p-nitrobenzoic acid and phthalic acid at a temperature of from -70 °C to the boiling point of the solvent used, generally from -50 °C to room temperature, preferably from -20 °C to 0 °C for about 5 minutes to 24 hours, preferably about 5 minutes to 20 hours, or in water or an alcohol solvent such as ethanol, methanol and propanol, in the presence of an alkali such as alkali hydroxide (e.g. sodium hydroxide, potassium hydroxide, calcium hydroxide) at a temperature of from -70 °C to the boiling point of the solvent used, generally from -50 °C to room temperature, preferably from -20 °C to 10 °C for about 5 minutes to 24 hours, preferably about 1 hour to 10 hours.

Method 2: A compound of the formula (I-2)

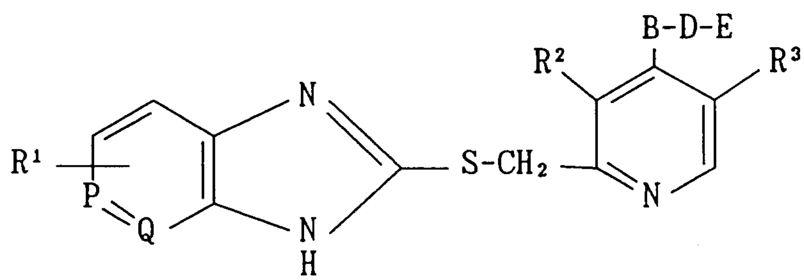

(I-2)

wherein each symbol is as defined above, is reacted with a compound of the formula (IV)

$R^9$ - NCX (IV)

wherein $R^9$ is sodium, potassium or alkyl and X is oxygen atom or sulfur atom, or a compound of the formula (V)

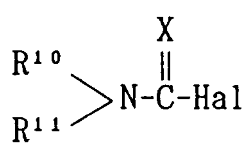

(V)

wherein $R^{10}$ and $R^{11}$ are alkyl, Hal is halogen and X is as defined above, in a suitable solvent such as acetic acid, tetrahydrofuran and acetonitrile to give a compound of the formula (I) wherein $R^4$ is carbamoyl, mono- or dialkylcarbamoyl, thiocarbamoyl or mono- or dialkylthiocarbamoyl and n is 0.

Method 3: A compound of the formula (I-2) is reacted with haloalkyl alcohol, alkoxyhaloalkyl, acyloxyhaloalkyl, alkyl, haloalkyl ester, haloalkylcarnoxylic acid, carbamoylhaloalkyl, or mono- or di-alkylcarbamoylhaloalkyl in the presence of an acid scavenger to give a compound of the formula (I) wherein $R^4$ is hydroxyalkyl, alkoxyalkyl, acyloxyalkyl, alkoxycarbonylalkyl, carboxyalykl, carbamoylalkyl or mono- or dialkylcarbamoylalkyl and n is 0.

Method 4: A compound of the formula (I-3)

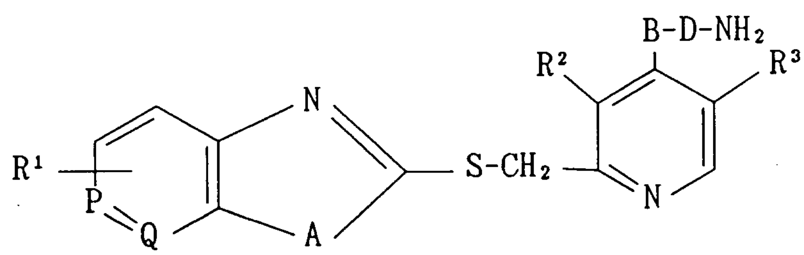

(I-3)

wherein each symbol is as defined above, is reacted with a compound of the formula (VII) or a compound of the formula (VI)

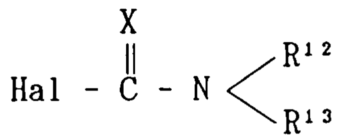

(VI)

wherein $R^{12}$ and $R^{13}$ are the same or different and each is hydrogen, alkyl, optionally substituted phenyl or optionally substituted phenylalkyl and Hal and X are as defined above, in a suitable solvent such as acetic acid, tetrahydrofuran and acetonitrile to give a compound of the formula (I) wherein $R^6$ and $R^7$ of E (a) are carbamoyl, mono- or dialkylcarbamoyl, optionally substituted phenylcarbamoyl, thiocarbamoyl, mono- or dialkylthiocarbamoyl, optionally substituted phenylthiocarbamoyl, optionally substituted phenylalkylcarbamoyl or optionally substituted phenylalkylthiocarbamoyl.
Method 5: A compound of the formula (I-4)

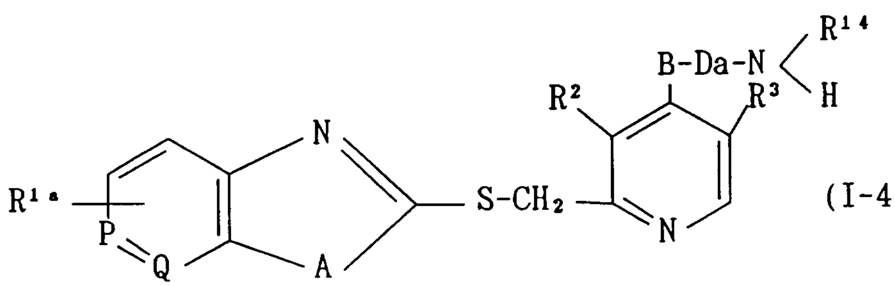

(I-4)

wherein $R^{1a}$ is hydrogen, halogen, alkyl, alkoxy, carboxyl, haloalkyl or nitro, Da is alkylene or alkylene substituted by carboxyl, $R^{14}$ is hydrogen, acyl or alkoxycarbonyl and other symbols are as defined above, is reacted with a compound of the formula (VII)

Hal - $R^{15}$ (VII)

wherein $R^{15}$ is alkyl, cycloalkyl, hydroxyalkyl, carboxyalkyl, optionally substituted phenylalkyl or optionally substituted heteroarylalkyl and Hal is as defined above, in a solvent inert to the reaction such as alcohol (e.g. methanol, ethanol), acetonitrile, tetrahydrofuran, benzene, toluene, dimethylformamide and a mixed solvent thereof, in the presence of an acid scavenger such as potassium carbonate, sodium carbonate, sodium methylate, sodium ethylate, sodium hydroxide, potassium tert-butoxide or triethylamine to give a compound of the formula (I-5)

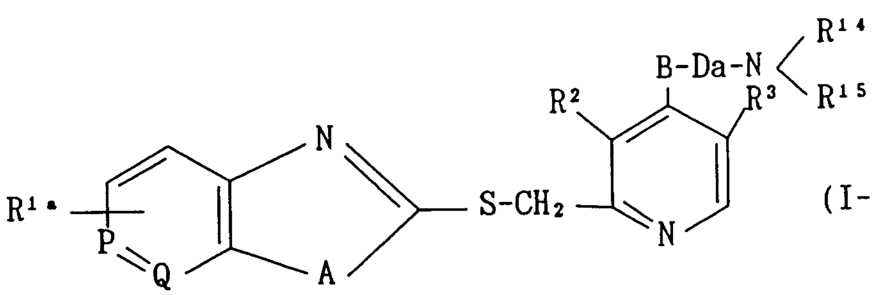

(I-5)

wherein each symbol is as defined above.
A compound of the formula (I-6)

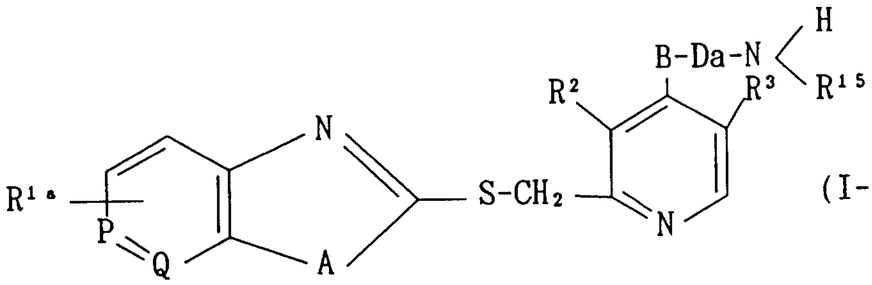

(I-6)

wherein each symbol is as defined above, can be obtained by hydrolyzing the compound of the formula (I-5) with an acid or alkali.

Further, a compound of the formula (I-7)

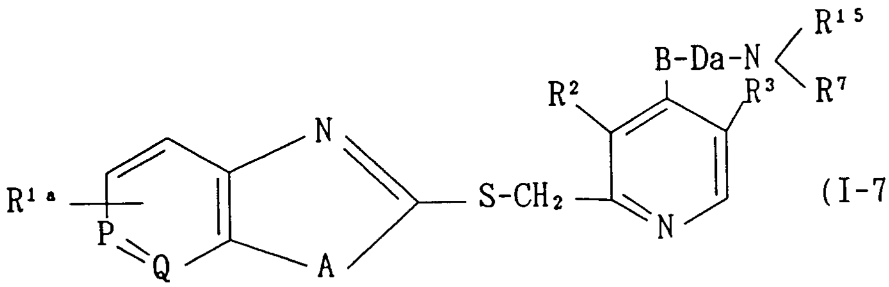

(I-7)

wherein each symbol is as defined above, can be obtained by reacting a compound of the formula (I-6) with a compound of the formula (VIII)

Hal - $R^7$ (VIII)

wherein each symbol is as defined above, in a solvent inert to the reaction, in the presence of an acid scavenger.

Method 6: A corresponding compound of the formula (I-1) having a carboxylic acid can be obtained by hydrolyzing a compound of the formula (I-1) having ester at the substituent $R^1$, A, D or E, with an acid or alkali.

The compound (I) thus obtained can be isolated and purified by a conventional method such as recrystallization and column chromatography.

The optical isomer of the compound (I) of the present invention can be produced by subjecting a reaction product to fractional crystallization or conducting the above-mentioned reactions using a starting compound optically resolved in advance.

The compound of the formula (I) of the present invention can be converted to the aforementioned acid addition salt by treating with hydrochloric acid, hydrobromic acid, hydroiodic acid, sulfuric acid, nitric acid, phosphoric acid, acetic acid, citric acid, maleic acid, fumaric acid, malonic acid, malic acid, tartaric acid, succinic acid, methanesulfonic acid or benzenesulfonic acid by a conventional method. Moreover, the compound of the formula (I') can be obtained by reacting with the corresponding salt.

The compound of the formula (III) can be produced, for example, by the following reactions.

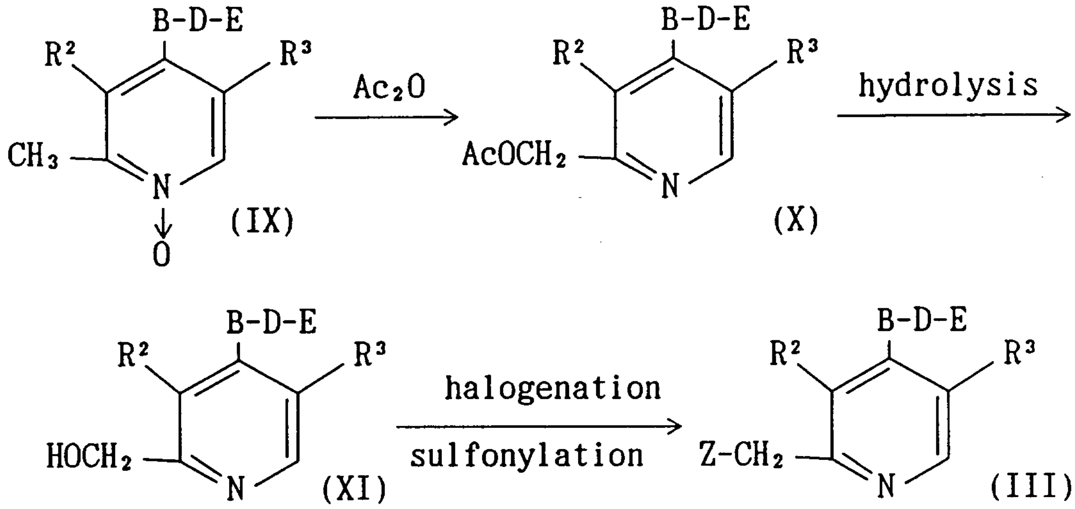


(IX) (X) (XI) (III)

wherein Ac is acetyl and other symbols are as defined above.

That is, a compound of the formula (IX) is heated with acetic anhydride to give a compound of the formula (X), which is hydrolyzed and treated with a halogenating agent such as thionyl chloride or with a sulfonylating agent such as methanesulfonyl chloride, whereby a compound of the formula (III) is produced.

A compound of the formula (IX) can be produced by the following reactions.

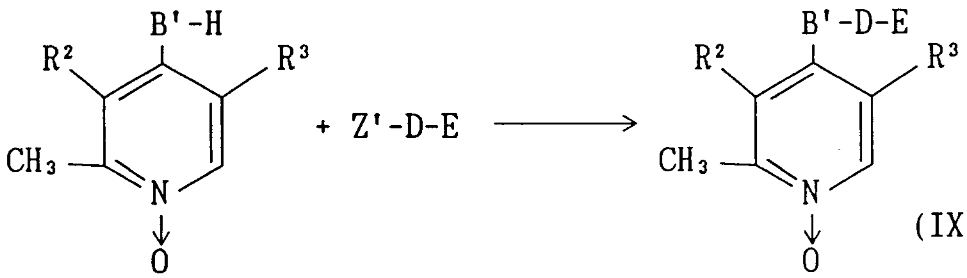

(IX')

wherein B' is a sulfur atom or N($R^5$), Z' is a leaving group such as chlorine, bromine, iodine, methanesulfonyloxy and p-toluenesulfonyloxy and other symbols are as defined above,

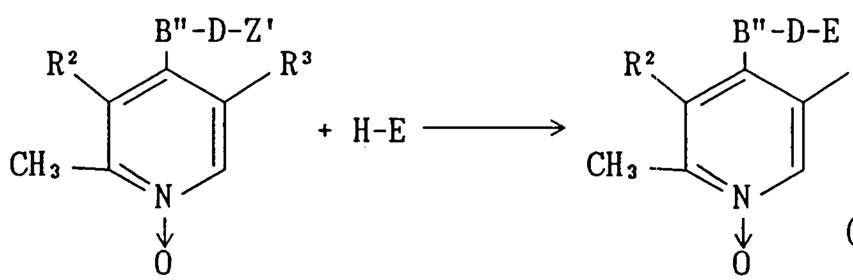

(IX")

wherein B" is an oxygen atom, a sulfur atom or N($R^5$) and other symbols are as defined above, and

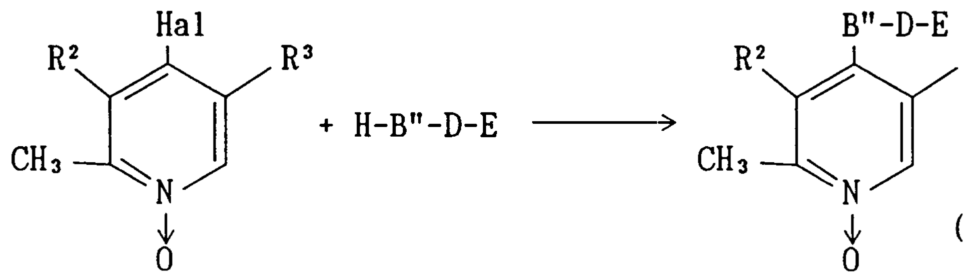

(IX")

wherein each symbol is as defined above.

The above-mentioned reactions proceed in a solvent inert to the reaction, such as water, alcohol (e.g. methanol, ethanol), chloroform, dichloromethane, benzene, toluene, dimethylformamide and a mixed solvent thereof, in the presence of a base such as sodium hydroxide, potassium hydroxide, sodium hydride, sodium methoxide, sodium ethoxide, sodium carbonate, potassium carbonate, metallic sodium, triethylamine and pyridine at a temperature of from about 0°C to the boiling point of the solvent used, preferably from 20°C to 80°C for about 10 minutes to 24 hours, preferably for 30 minutes to 7 hours.

The compound of the formula (IX) wherein B is SO or $SO_2$ can be produced by subjecting a compound of the formula (IX) wherein B is a sulfur atom to oxidation. The oxidizing agent to be used for the oxidation includes, for example, metachloroperbenzoic acid, peracetic acid, trifluoroperacetic acid, permaleic acid, potassium hyperoxide, sodium bromite, sodium hypobromite and hydrogen peroxide. The reaction is carried out in a solvent inert to the reaction, such as water, dichloromethane, chloroform, tetrahydrofuran, dioxane, dimethylformamide and a mixed solvent thereof, in the presence of an organic acid such as formic acid, acetic acid, propionic acid, butyric acid, maleic acid, fumaric acid, malonic acid, succinic acid, benzoic acid, metachlorobenzoic acid, p-nitrobenzoic acid and phthalic acid, at a temperature of from -70°C to the boiling point of the solvent used, generally from -50°C to the room temperature, preferably from -20°C to 0°C for about 5 minutes to 24 hours, preferably for about 5 minutes to 20 hours, or in water or an alcohol solvent such as ethanol, methanol and propanol, in the presence of an alkali such as alkali hydroxide (e.g. sodium hydroxide, potassium hydroxide, calcium hydroxide) at a temperature of from -70°C to the boiling point of the solvent used, generally from -50°C to the room temperature, preferably from -20°C to 10°C for about 5 minutes to 24 hours, preferably for 1 hour to 10 hours.

The compounds of the present invention show selective antibacterial activity against the bacteria belonging to the genus *Helicobacter* represented by *Helicobacter pylori* and are useful for the prevention and treatment of various diseases caused by *Helicobacter*. That is, the compounds of the present invention are usable for mammals inclusive of human for the prevention and treatment of various diseases caused by the bacteria belonging to the genus *Helicobacter*, the prevention of recurrence and relapse of ulcer, suppression of vomiting, the prevention and treatment of non-ulcer dyspepsia and the prevention and treatment of ulcer. In addition, the compounds of the present invention have antiulcer activity, gastric acid secretion-suppressive activity, gastrointestinal cytoprotective activity and anti-diarrhea activity and are useful for the prevention and treatment of gastrointestinal diseases such as gastric ulcer, duodenal ulcer, gastritis, diarrhea and colitis. The compounds of the present invention have low toxicity, are stable to acid etc. and show small increase in gastrin value in blood. Such pharmacological action of the compounds of the present invention can be confirmed by the method of Ghosh et al, Br. J. Pharmacol. *13*, p 54 (1958). The compounds of the present invention can be used for the treatment of osterocacochymia and glaucoma, as well as for the prevention and treatment of inflammatory diseases such as articular rheumatism and gout.

Experimental Example 1

Antibacterial activity (*in vitro*) of the compound of the present invention against *Helicobacter pylori* was determined by the following agar plate dilution method.

Clinical isolates (18 strains) were inoculated on Brucella agar supplemented with 5% horse serum and incubated in a microaerophilic environment at 37°C for 72 hours. The cultures were diluted with Brucella broth to give a concentration of approximately $10^6$ cell/ml. The diluted bacterial solution was spot inoculated with a microplanter on an agar plate containing 2-fold dilution concentration series of the test compound. The cells were incubated at 37°C under 8% $CO_2$ for 3-4 days. The minimum inhibitory concentration (MIC) in respective strains was determined, based on which $MIC_{50}$ and $MIC_{90}$ were calculated. The results are shown in Table 1.

Table 1

| Test compound (Example No.) | $MIC_{50}$ (μg/ml) | $MIC_{90}$ (μg/ml) |
|---|---|---|
| 1 | ≦ 0.006 | 0.012 |
| 2 | ≦ 0.006 | ≦ 0.006 |
| 3 | 0.025 | 0.05 |
| 7 | 0.012 | 0.025 |
| 12 | 0.012 | 0.025 |
| 28 | 0.025 | 0.025 |
| Ref. Compound 1 | 0.05 | 0.10 |
| Ref. Compound 2 | 0.10 | 0.20 |
| Ref. Compound 3 | 0.05 | 0.10 |

Reference Compound 1: 2-(3-methyl-4-(2-(1,2,3,4-tetrahydroisoquinolin-2-yl)ethoxy)-2-pyridyl)methylthio-1H-benzimidazole (Compound 16 of WO92/12976)
Reference Compound 2: 2-(3-methyl-4-(2-(N-benzyl-N-methylamino)ethoxy)-2-pyridyl)methylsulfinyl-1H-benzimidazole 1/2 magnesium salt (Compound 17 of WO92/12976)
Reference Compound 3: 2-(3-methyl-4-(2-(1,2,3,4-tetrahydroisoquinolin-2-yl)ethoxy)-2-pyridyl)methylsulfinyl-1H-benzimidazole 1/2 magnesium salt (Compound 20 of WO92/12976)

Experimental Example 2

The test bacterium precultured in Muller-Hinton liquid medium was diluted with the same medium to give a bacterial solution of about $10^6$ cell/ml for inoculation. The dilute bacterial solution was spot inoculated with a microplanter on an agar plate containing 2-fold dilution concentration series of the test compound and the cells were incubated at 37°C under 8% $CO_2$ for 3-4 days. The minimum inhibitory concentration (MIC) in respective strains was determined, based on which MIC was calculated. The results are shown in Table 2.

Table 2

| Test compound (Example No.) | MIC (μg/ml) | | | |
|---|---|---|---|---|
| | *S. aureus* | *E. coli* | *P. aeruginosa* | *C. albicans* |
| 1 | >100 | >100 | >100 | >100 |
| 2 | >100 | >100 | >100 | >100 |
| 3 | >100 | >100 | >100 | >100 |
| 7 | >100 | >100 | >100 | >100 |
| 28 | >100 | >100 | >100 | >100 |

*S. aureus* : *Staphylococcus aureus* FDA 209P
*E. coli* : *Escherichia coli* NIHJ JC-2
*P. aeruginosa* : *Pseudomonas aeruginosa* IFO 12582
*C. albicans* : *Candida albicans* IFO 1060

As is evident from Table 2, the compound of the formula (I) was ineffective against Gram positive bacteria, Gram negative bacteria and fungi.

Experimental Example 3

Male rats weighing about 200 g (16 rats per group) were fasted for 20 hours and the test solution was orally administered to the rats. Thirty minutes later, aspirin (100 mg/kg) containing 0.3N hydrochloric acid was orally administered.

Four hours later, the stomach was removed and the area which developed ulcer was examined. A square root of the total ulcer area was taken as an ulcer index.

Table 3

| Test compound (Example No.) | Dose (mg/kg p.o.) | Ulcer index (mean±standard deviation) | Change (%) |
|---|---|---|---|
| Control | | 5.0±0.51 | |
| 1 | 10 | 3.1±0.33** | -37 |
| | 30 | 2.3±0.49** | -55 |

Experimental Example 4

The compound of Example 2 was orally administered to male ddY mice by 1000 mg/kg or intraperitoneally by 300 mg/kg and the mice were observed for 5 days. As a result, no death case was found.

When the compound of the present invention is used as a pharmaceutical preparation, a therapeutically effective amount of Compound (I) or a pharmaceutically acceptable salt thereof is admixed with pharmaceutically acceptable additives such as excipients, carriers, diluents and solubilizers and formulated into capsules, tablets (inclusive of sugar-coated tablets and film-coated tablets), granules, injections or infusions for administration. The dose is generally about 0.01-30 mg/kg, preferably 0.1-3 mg/kg daily for an adult by oral administration, though it is subject to change depending on the symptom, age, resistance to drug etc. of the patients.

Best Mode for Embodying the Invention

The present invention is explained in more detail by illustrating Reference Examples and Examples, to which the invention is not limited.

Reference Example 1

4-(2-Chloroethylthio)-2,3-dimethylpyridine-N-oxide (6 g) was dissolved in a mixed solution of dimethylformamide-toluene (1:1) and thereto were added potassium carbonate (4.3 g), potassium iodide (4.4 g) and morpholine (1.9 ml). The mixture was reacted at 50-55 °C for 12 hours. After the completion of the reaction, inorganic matters were filtered off and the solvent was distilled away. Water was added to the residue and the mixture was extracted with chloroform. After drying over anhydrous magnesium sulfate, the chloroform was distilled away and the obtained residue was purified by silica gel column chromatography to give 3.4 g of 2,3-dimethyl-4-(2-morpholinoethylthio)pyridine-N-oxide.

$^1$H-NMR ($CDCl_3$): 2.34 (s,3H), 2.55 (s,3H), 2.44-2.60 (m,4H), 2.71 (t,2H), 3.09 (t,2H), 3.64-3.80 (m,4H), 6.96 (d,1H), 8.07 (d,1H)

Reference Example 2

2,3-Dimethyl-4-(2-(1,2,3,4-tetrahydroisoquinolin-2-yl)ethylthio)pyridine-N-oxide

$^1$H-NMR ($CDCl_3$): 2.32 (s,3H), 2.52 (s,3H), 2.70-3.00 and 3.08-3.29 (m,8H), 3.70 (s,2H), 7.00 (d,1H), 7.02-7.20 (m,4H), 8.05 (d,1H)

Reference Example 3

4-(2-(N-Benzyl-N-methylamino)ethylthio)-2,3-dimethylpyridine-N-oxide

$^1$H-NMR ($CDCl_3$): 2.28 (s,3H), 2.31 (s,3H), 2.53 (s,3H), 2.70 (t,2H), 3.04 (t,2H), 3.58 (s,2H), 6.76 (d,1H), 7.26 (s,5H), 7.93 (d,1H)

Reference Example 4

2,3-Dimethyl-4-(2-piperidinoethylthio)pyridine-N-oxide

Reference Example 5

2,3-Dimethyl-4-(2-morpholinoethylthio)pyridine-N-oxide (3.0 g) was dissolved in acetic anhydride (30 ml) and the mixture was stirred at 90°C for 1.5 hours. The solvent was distilled away and water was added to the residue. The mixture was extracted with chloroform and dried over anhydrous magnesium sulfate, and the solvent was distilled away. The residual 2-acetoxymethyl-3-methyl-4-(2-morpholinoethylthio)pyridine (3.2 g) was dissolved in methanol (30 ml) and thereto was added sodium hydroxide (0.88 g) in water (10 ml) under ice-cooling. The mixture was stirred under ice-cooling for 1.5 hours and at room temperature for 2 hours. After the completion of the reaction, the methanol was distilled away and the residue was extracted with chloroform. The chloroform layer was dried over anhydrous magnesium sulfate and the solvent was distilled away to give 2.1 g of 2-hydroxymethyl-3-methyl-4-(2-morpholinoethylthio)pyridine.

$^1$H-NMR ($CDCl_3$): 2.36 (s,3H), 2.47-2.67 (m,4H), 2.75 (t,2H), 3.15 (t,2H), 3.68-3.87 (m,4H), 4.68 (s,2H), 7.05 (d,1H), 8.23 (d,1H)

The following compounds were obtained in the same manner.

Reference Example 6

2-Hydroxymethyl-3-methyl-4-(2-(1,2,3,4-tetrahydroisoquinolin-2-yl)ethylthio)pyridine

Reference Example 7

4-(2-(N-Benzyl-N-methylamino)ethylthio)-2-hydroxymethyl-3-methylpyridine

$^1$H-NMR ($CDCl_3$): 2.18 (s,3H), 2.33 (s,3H), 2.81 (t,2H), 3.10 (t,2H), 3.60 (s,2H), 4.81 (s,2H), 6.92 (d,1H), 7.30 (s,5H), 8.21 (d,2H)

Reference Example 8

2-Hydroxymethyl-3-methyl-4-(2-piperidinoethylthio)pyridine

Example 1

Thionyl chloride (20 ml) was dropwise added to 2-hydroxymethyl-3-methyl-4-(2-morpholinoethylthio)-pyridine (2 g) under ice-cooling and the mixture was stirred at room temperature for 1.5 hours. After the completion of the reaction, the thionyl chloride was distilled away. The residue was alkali-oversaturated with potassium carbonate and extracted with chloroform. The chloroform layer was dried over anhydrous magnesium sulfate and the solvent was distilled away to give 2.1 g of 2-chloromethyl-3-methyl-4-(2-morpholinoethylthio)pyridine as an oily substance. The oily substance was added to ethanol (50 ml) containing a solution of 2-mercaptobenzimidazole (1.0 g) and sodium hydroxide (0.6 g) in water (10 ml) and the mixture was refluxed under heating for 5 hours. After the completion of the reaction, the ethanol was distilled away and water was added to the residue, followed by extraction with chloroform. The extract was dried over anhydrous magnesium sulfate and the solvent was distilled away. Acetone was added to the residue to allow crystallization. The obtained crystals were recrystallized from a mixed solvent of ethanol-acetone to give 2-(3-methyl-4-(2-morpholinoethylthio)-2-pyridyl)methylthio-1H-benzimidazole as white crystals, melting point 201-203°C.

$^1$H-NMR ($CDCl_3$): 2.37 (s,3H), 2.43-2.60 (m,4H), 2.70 (t,2H), 3.10(t,2H), 3.72 (m,4H), 4.41 (s,2H), 7.08 (d,1H), 8.29 (d,1H), 7.08-7.26 and 7.45-7.56 (m,4H)

Example 2

Thionyl chloride (20 ml) was dropwise added to 2-hydroxymethyl-3-methyl-4-(2-(1,2,3,4-tetrahydroisoquinolin-2-yl)ethylthio)pyridine (2.8 g) under ice-cooling and the mixture was stirred at room temperature for 1.5 hours. After the completion of the reaction, the thionyl chloride was distilled away. The residue was alkali-oversaturated with potassium carbonate and extracted with chloroform. The chloroform

layer was dried over anhydrous magnesium sulfate and the solvent was distilled away to give 2.1 g of 2-chloromethyl-3-methyl-4-(2-(1,2,3,4-tetrahydroisoquinolin-2-yl)ethylthio)pyridine. The product was added to ethanol (30 ml) containing a solution of 2-mercaptobenzimidazole (1.3 g) and sodium hydroxide (1.2 g) in water (10 ml) and the mixture was refluxed under heating for 2 hours. After the completion of the reaction, the ethanol was distilled away and water was added to the residue, followed by extraction with chloroform. The extract was dried over anhydrous magnesium sulfate and the solvent was distilled away. Acetone was added to the residue to allow crystallization. The obtained crystals were recrystallized from isopropyl alcohol to give 2-(3-methyl-4-(2-(1,2,3,4-tetrahydroisoquinolin-2-yl)ethylthio)-2-pyridyl)methylthio-1H-benzimidazole as crystals, melting point 90-94 °C.

$^1$H-NMR ($CDCl_3$): 2.38 (s,3H), 2.94 (t,2H), 2.94 (s,4H), 3.26 (t,2H), 3.84 (s,2H), 4.43 (s,2H), 7.00-7.60 (m, 9H), 8.30 (d,1H)

Example 3

Thionyl chloride (10 ml) was dropwise added to 4-(2-(N-benzyl-N-methylamino)ethylthio)-2-hydroxymethyl-3-methylpyridine (1.8 g) under ice-cooling and the mixture was stirred at room temperature for 1.5 hours. After the completion of the reaction, thionyl chloride was distilled away. The residue was alkali-oversaturated with potassium carbonate and extracted with chloroform. The chloroform layer was dried over anhydrous magnesium sulfate and the solvent was distilled away to give 1.7 g of 4-(2-(N-benzyl-N-methylamino)ethylthio)-2-chloromethyl-3-methylpyridine. The product was added to ethanol (30 ml) containing a solution of 2-mercaptobenzimidazole (0.9 g) and sodium hydroxide (0.5 g) in water (10 ml) and the mixture was refluxed under heating for 7 hours. After the completion of the reaction, the ethanol was distilled away and water was added to the residue, followed by extraction with chloroform. The extract was dried over anhydrous magnesium sulfate and the solvent was distilled away. Acetone was added to the residue to allow crystallization. The obtained crystals were recrystallized from a mixed solvent of acetone-isopropyl ether to give 2-4-(2-(N-benzyl-N-methylamino)ethylthio)-3-methyl-2-pyridyl)methylthio-1H-benzimidazole as crystals, melting point 110-112 °C.

$^1$H-NMR ($CDCl_3$): 2.31 (s,6H), 2.76 (t,2H), 3.10 (t,2H), 3.60 (s,2H), 4.40 (s,2H), 6.90 (d,1H), 7.10-7.58 (m,9H), 8.20 (d,1H)

Example 4

Thionyl chloride (15 ml) was dropwise added to 2-hydroxymethyl-3-methyl-4-(2-piperidinoethylthio)-pyridine (2.5 g) under ice-cooling and the mixture was stirred at room temperature for 3 hours. After the completion of the reaction, thionyl chloride was distilled away. The residue was alkali-oversaturated with potassium carbonate and extracted with chloroform. The chloroform layer was dried over anhydrous magnesium sulfate and the solvent was distilled away to give 2.0 g of 2-chloromethyl-3-methyl-4-(2-piperidinoethylthio)pyridine as an oily substance. The substance was added to ethanol (50 ml) containing a solution of 2-mercaptobenzimidazol e (1.0 g) and sodium hydroxide (0.6 g) in water (10 ml) and the mixture was refluxed under heating for 5 hours. After the completion of the reaction, the ethanol was distilled away and water was added to the residue, followed by extraction with chloroform. The extract was dried over anhydrous magnesium sulfate and the solvent was distilled away. The residue was recrystallized to give 2-(3-methyl-4-(2-piperidinoethylthio)-2-pyridyl)methylthio-1H-benzimidazole, melting point 138-140 °C.

Example 5

The same reaction as in Example 1 was carried out using 4-(2-(2-dimethylaminomethylmorpholino)-ethylthio)-2-hydroxymethyl-3-methylpyridine in place of 2-hydroxymethyl-3-methyl-4-(2-morpholinoethylthio)pyridine to give 2-(4-(2-(2-dimethylaminomethylmorpholino)ethylthio)-3-methyl-2-pyridyl)methylthio-1H-benzimidazole.

$^1$H-NMR ($CDCl_3$): 1.72-2.88 (m,8H), 2.26 (s,6H), 2.35 (s,3H), 3.09 (t,2H), 3.50-4.00 (m,3H), 4.44 (s,2H), 7.00-7.28 and 7.30-7.60 (m,5H), 8.20 (d,1H)

Example 6

The same reaction as in Example 1 was carried out using 2-hydroxymethyl-3-methyl-4-(2-(2-dimethylaminomethylmorpholino)-2-oxoethylthio)pyridine in place of 2-hydroxymethyl-3-methyl-4-(2-morpholinoethylthio)pyridine to give 2-(3-methyl-4-(2-(2-dimethylaminomethylmorpholino)-2-oxoethylthio)-2-pyridyl)methylthio-1H-benzimidazole 3/2 hydrate, melting point 160 ° C.

Example 7

The same reaction as in Example 1 was carried out using 4-(1-benzyl-4-piperidyl)thio-2-hydroxymethyl-3-methylpyridine in place of 2-hydroxymethyl-3-methyl-4-(2-morpholinoethylthio)pyridine, followed by treatment with ethanolic hydrochloric acid to give 2-(4-(1-benzyl-4-piperidyl)thio-3-methyl-2-pyridyl)methylthio-1H-benzimidazole hydrochloride hydrate, melting point 168 ° C (dec.).

Example 8

The same reaction as in Example 1 was carried out using 4-(2-(4-acetylpiperazin-1-yl)ethylthio)-2-hydroxymethyl-3-methylpyridine in place of 2-hydroxymethyl-3-methyl-4-(2-morpholinoethylthio)pyridine to give 2-(3-methyl-4-(2-(4-acetylpiperazin-1-yl)ethylthio)-2-pyridyl)methylthio-1H-benzimidazole 1/2 hydrate, melting point 129-131 ° C.

Example 9

The same reaction as in Example 1 was carried out using 2-hydroxymethyl-3-methyl-4-(2-acetylaminoethylthio)pyridine in place of 2-hydroxymethyl-3-methyl-4-(2-morpholinoethylthio)pyridine to give 2-(3-methyl-4-(2-acetylaminoethylthio)-2-pyridyl)methylthio-1H-benzimidazole 1/4 ethanol, melting point 211-213 ° C.

Example 10

The same reaction as in Example 1 was carried out using 2-hydroxymethyl-3-methyl-4-(3-morpholinopropylthio)pyridine in place of 2-hydroxymethyl-3-methyl-4-(2-morpholinoethylthio)pyridine to give 2-(3-methyl-4-(3-morpholinopropylthio)-2-pyridyl)methylthio-1H-benzimidazole as crystals, melting point 149-150 ° C.

Example 11

The same reaction as in Example 1 was carried out using 2-mercaptobenzothiazole in place of 2-mercaptobenzimidazole to give 2-((3-methyl-4-(2-morpholinoethylthio)-2-pyridyl)methylthio)benzothiazole as crystals, melting point 91-93 ° C.

Example 12

The same reaction as in Example 1 was carried out using 2-hydroxymethyl-3-methyl-4-(2-(1,2,3,4-tetrahydroisoquinlin-2-yl)ethylthio)pyridine in place of 2-hydroxymethyl-3-methyl-4-(2-morpholinoethylthio)-pyridine and 2-mercaptoimidazo[5,4-b]pyridine in place of 2-mercaptobenzimidazole to give 2-((3-methyl-4-(2-(1,2,3,4-tetrahydroisoquinolin-2-yl)ethylthio)-2-pyridyl)methylthio)imidazo[5,4-b]pyridine as crystals, melting point 180 ° C.

Example 13

The same reaction as in Example 1 was carried out using 2-hydroxymethyl-3-methyl-4-(2-(1,2,3,4-tetrahydroisoquinolin-2-yl)ethylthio)pyridine in place of 2-hydroxymethyl-3-methyl-4-(2-morpholinoethylthio)-pyridine and 2-mercaptoimidazo[5,4-c]pyridine in place of 2-mercaptobenzimidazole to give 2-((3-methyl-4-(2-(1,2,3,4-tetrahydroisoquinolin-2-yl)ethylthio)-2-pyridyl)methylthio)imidazo[5,4-c]pyridine as crystals, melting point 215 ° C (dec.).

Example 14

The same reaction as in Example 1 was carried out using 2-hydroxymethyl-4-(2-(1,2,3,4-tetrahydroisoquinlin-2-yl)ethylthio)pyridine in place of 2-hydroxymethyl-3-methyl-4-(2-morpholinoethylthio)-pyridine to give 2-(4-(2-(1,2,3,4-tetrahydroisoquinolin-2-yl)ethylthio)-2-pyridyl)methylthio-1H-benzimidazole as crystals, melting point 125-127 ° C.

Example 15

2-(3-Methyl-4-(2-(4-acetylpiperazin-1-yl)ethylthio)-2-pyridyl)methylthio-1H-benzimidazole (38 g) was dissolved in 6N hydrochloric acid and the mixture was stirred at 50°C for 2 hours. After the completion of the reaction, the mixture was made alkaline with potassium carbonate and extracted with a mixed solvent of chloroform-methanol. The chloroform-methanol layer was dried over anhydrous magnesium sulfate, the solvent was distilled away and the residue was recrystallized from ethanol to give 2-(3-methyl-4-(2-(piperazin-1-yl)ethylthio)-2-pyridyl)methylthio-1H-benzimidazole as crystals, melting point 166-168°C.

Example 16

The same reaction as in Example 15 was carried out using 2-(3-methyl-4-(2-acetylaminoethylthio)-2-pyridyl)methylthio-1H-benzimidazole in place of 2-(3-methyl-4-(2-(4-acetylpiperazin-1-yl)ethylthio)-2-pyridyl)-methylthio-1H-benzimidazole to give 2-(3-methyl-4-(2-aminoethylthio)-2-pyridyl)methylthio-1H-benzimidazole 1/5 hydrate, melting point 158-160°C.

Example 17

2-(3-Methyl-4-(2-morpholinoethylthio)-2-pyridyl)methylthio-1H-benzimidazole (10 g) was reacted with 1.1 equivalents of ethyl chlorocarbonate in dimethylformamide in the presence of 1.1 equivalents of sodium hydride at room temperature. After the completion of the reaction, the solvent was distilled away and water was added to the residue, followed by extraction with chloroform. The chloroform layer was dried over anhydrous magnesium sulfate and the solvent was distilled away to give 11 g of ethyl 2-(3-methyl-4-(2-morpholinoethylthio)-2-pyridyl)methylthio-1H-benzimidazole-1-carboxylate as crystals, melting point 145-147°C.

Example 18

The same reaction as in Example 17 was carried out using chloroacetic acid in place of ethyl chlorocarbonate to give 2-(3-methyl-4-(2-morpholinoethylthio)-2-pyridyl)methylthio-1H-benzimidazole-1-acetic acid as crystals, melting point 223-225°C.

Example 19

The same reaction as in Example 17 was carried out using methyl chlorocarbonate in place of ethyl chlorocarbonate to give methyl 2-(3-methyl-4-(2-morpholinoethylthio)-2-pyridyl)methylthio-1H-benzimidazole-1-carboxylate as crystals, melting point 151-153°C.

Example 20

The same reaction as in Example 17 was carried out using ethylene bromohydrin in place of ethyl chlorocarbonate to give 2-(2-(3-methyl-4-(2-morpholinoethylthio)-2-pyridyl)methylthio-1H-benzimidazol-1-yl)-ethanol as crystals, melting point 141-143°C.

Example 21

The same reaction as in Example 17 was carried out using methyl chloroacetate in place of ethyl chlorocarbonate to give methyl 2-(3-methyl-4-(2-morpholinoethylthio)-2-pyridyl)methylthio-1H-benzimidazole-1-acetate as crystals, melting point 128-129°C.

Example 22

The same reaction as in Example 17 was carried out using dimethylcarbamyl chloride in place of ethyl chlorocarbonate to give N,N-dimethyl-2-(3-methyl-4-(2-morpholinoethylthio)-2-pyridyl)methylthio-1H-benzimidazole-1-acetamide as crystals, melting point 178-180°C.

Example 23

The same reaction as in Example 17 was carried out using dimethylcarbamylmethyl chloride in place of ethyl chlorocarbonate to give 1-(N,N-dimethylcarbamoyl)-2-(3-methyl-4-(2-morpholinoethylthio)-2-pyridyl)-methylthio-1H-benzimidazole 1/4 hydrate as crystals, melting point 117-119 ° C.

Example 24

The same reaction as in Example 17 was carried out using methyl iodide in place of ethyl chlorocarbonate, followed by treatment with ethanolic hydrochloric acid to give 1-methyl-2-(3-methyl-4-(2-morpholinoethylthio)-2-pyridyl)methylthio-1H-benzimidazole hydrochloride 1/5 hydrate as crystals, melting point 214-216 ° C.

Example 25

The same reaction as in Example 17 was carried out using 2-chloroacetamide in place of ethyl chlorocarbonate to give 2-(3-methyl-4-(2-morpholinoethylthio)-2-pyridyl)methylthio-1H-benzimidazole-1-acetamide 1/5 hydrate as crystals, melting point 178-180 ° C.

Example 26

The same reaction as in Example 17 was carried out except that 2-(3-methyl-4-(2-(1,2,3,4-tetrahydroisoquinolin-2-yl)ethylthio)-2-pyridyl)methylthio-1H-benzimidazole was reacted with ethyl chlorocarbonate, in place of 2-(3-methyl-4-(2-morpholinoethylthio)-2-pyridyl)methylthio-1H-benzimidazole, to give ethyl 2-(3-methyl-4-(2-(1,2,3,4-tetrahydroisoquinolin-2-yl)ethylthio)-2-pyridyl)methylthio-1H-benzimidazole-1-carboxylate 1/2• ethanol as crystals, melting point 134-136 ° C (dec.).

Example 27

2-Hydroxymethyl-3-methyl-4-(2-morpholinoethylthio)pyridine (6.2 g) was dissolved in chloroform and thereto was dropwise added thionyl chloride (2 ml) under ice-cooling. The mixture was stirred at room temperature for 1 hour. After the completion of the reaction, the reaction mixture was poured into ice-water and made alkaline with potassium carbonate, followed by extraction with chloroform. The chloroform layer was dried over anhydrous magnesium sulfate and the solvent was distilled away to give 6.0 g of 2-chloromethyl-3-methyl-4-(2-morpholinoethylthio)pyridine as an oily substance. The substance was added to ethanol (60 ml) containing a solution of 5-methoxycarbonyl-2-mercaptobenzimidazole (5.1 g) and sodium hydroxide (1.6 g) in water (10 ml) and the mixture was refluxed under heating for 3.5 hours. After the completion of the reaction, the ethanol was distilled away and the residue was dissolved in water. The aqueous layer was washed with chloroform and dilute hydrochloric acid was dropwise added until crystals precipitated in the aqueous layer. The resultant crystals were collected by filtration to give 2-(3-methyl-4-(2-morpholinoethylthio)-2-pyridyl)methylthio-1H-benzimidazole-5-carboxylic acid 7/2 hydrate, melting point 120-124 ° C.

Example 28

2-(3-Methyl-4-(2-morpholinoethylthio)-2-pyridyl)methylthio-1H-benzimidazole (3 g) was dissolved in chloroform and thereto was added meta-chloroperbenzoic acid (1.9 g) at -50 ° C. The mixture was stirred at -30 ° C for 5 hours. After the completion of the reaction, an aqueous solution of sodium thiosulfate was added thereto and the mixture was stirred. The chloroform layer was partitioned and dried over anhydrous magnesium sulfate. The chloroform was distilled away. The residue was purified by column chromatography to give 4-(2-((2-(1H-benzimidazol-2-yl)thiomethyl-3-methyl-4-pyridyl)thio)ethyl)morpholine N-oxide 1/2 hydrate as crystals, melting point 141-143 ° C.

Example 29

2-(3-Methyl-4-(2-aminoethylthio)-2-pyridyl)methylthio-1H-benzimidazole (8.5 g) was dissolved in ethanol and thereto was added benzaldehyde (2.8 g). The mixture was stirred at 50-55 ° C for 5 hours and sodium borohydride (0.98 g) was added thereto under ice-cooling. The mixture was stirred at room temperature for

2 hours. After the completion of the reaction, ethanol was distilled away and the residue was eluted with chloroform. The chloroform layer was dried over anhydrous magnesium sulfate and the solvent was distilled away. The residue was purified by column chromatography to give 2-(3-methyl-4-(2-benzylaminoethylthio)-2-pyridyl)methylthio-1H-benzimidazole.

$^1$H-NMR ($CDCl_3$): 2.33 (s,3H), 3.00 (m,4H), 3.80 (s,2H), 4.40 (s,2H), 7.00 and 8.18 (d,2H), 7.05-7.51 (m,9H)

Example 30

2-(3-Methyl-4-(2-benzylaminoethylthio)pyridin-2-yl)methylthio-1H-benzimidazole (2 g) was dissolved in dimethylformamide and reacted with methyl chloroacetate (0.45 ml) in the presence of potassium carbonate (0.71 g) at 50-60 °C. After the completion of the reaction, the dimethylformamide was distilled away and the residue was extracted with ethyl acetate. The ethyl acetate layer was dried over anhydrous magnesium sulfate and the solvent was distilled away to give an oily product. The oily product was purified by column chromatography to give 2-(3-methyl-4-(2-(N-benzyl-N-methoxycarbonylmethylamino)ethylthio)-2-pyridyl)-methylthio-1H-benzimidazole.

Example 31

2-(3-Methyl-4-(2-benzylaminoethylthio)-2-pyridyl)methylthio-1H-benzimidazole (1.88 g) was dissolved in dimethylformamide (20 ml) and thereto were added ethyl bromoacetate (0.784 g) and potassium carbonate (0.925 g). The mixture was stirred at 40-50 °C for 1 hour. The reaction mixture was poured into water and extracted with ethyl acetate. The extract was washed with water, dried and concentrated under reduced pressure. The residue was subjected to column chromatography and eluted with chloroform-methanol to give 2-(3-methyl-4-(2-(N-benzyl-N-ethoxycarbonylmethyl)aminoethylthio)-2-pyridyl)methylthio-1H-benzimidazole as pale yellow crystals.

The compounds of Examples 32-40 can be synthesized using 2-(3-methyl-4-(2-benzylaminoethylthio)-2-pyridyl)methylthio-1H-benzimidazole as a starting compound.

Example 32

2-(3-methyl-4-(2-(N-benzyl-N-ethoxycarbonylamino)ethylthio)-2-pyridyl)methylthio-1H-benzimidazole

Example 33

2-(3-methyl-4-(2-(N-benzyl-N-(2-ethoxycarbonylethyl)amino)ethylthio)-2-pyridyl)methylthio-1H-benzimidazole

Example 34

2-(3-methyl-4-(2-(N-benzyl-N-(3-ethoxycarbonylpropyl)amino)ethylthio)-2-pyridyl)methylthio-1H-benzimidazole

Example 35

2-(3-methyl-4-(2-(N-benzyl-N-(4-ethoxycarbonylbutyl)amino)ethylthio)-2-pyridyl)methylthio-1H-benzimidazole

Example 36

2-(3-methyl-4-(2-(N-benzyl-N-(5-ethoxycarbonylpentyl)amino)ethylthio)-2-pyridyl)methylthio-1H-benzimidazole

Example 37

2-(3-methyl-4-(2-(N-benzyl-N-(2-hydroxyethyl)amino)ethylthio)-2-pyridyl)methylthio-1H-benzimidazole

Example 38

2-(3-methyl-4-(2-(N-benzyl-N-(3-hydroxypropyl)amino)ethylthio)-2-pyridyl)methylthio-1H-benzimidazole

Example 39

2-(3-methyl-4-(2-(N-benzyl-N-(4-hydroxybutyl)amino)ethylthio)-2-pyridyl)methylthio-1H-benzimidazole

Example 40

2-(3-methyl-4-(3-(N-benzyl-N-(2,3-dihydroxypropyl)amino)ethylthio)-2-pyridyl)methylthio-1H-benzimidazole

Example 41

2-(3-Methyl-4-(3-benzylaminopropylthio)-2-pyridyl)methylthio-1H-benzimidazole (2.0 g) was dissolved in dimethylformamide (25 ml) and thereto were added ethyl bromoacetate (0.834 g) and potassium carbonate (0.984 g). The mixture was stirred at 40-50 °C for 1 hour. The reaction mixture was poured into water and extracted with ethyl acetate. The extract was washed with water, dried and concentrated under reduced pressure. The residue was subjected to column chromatography and eluted with chloroform-methanol to give 2-(3-methyl-4-(3-(N-benzyl-N-ethoxycarbonylmethylamino)propylthio)-2-pyridyl)methylthio-1H-benzimidazole.

The compounds of Examples 42-50 can be synthesized using 2-(3-methyl-4-(3-benzylaminopropylthio)-2-pyridyl)methylthio-1H-benzimidazole as a starting compound.

Example 42

2-(3-methyl-4-(3-(N-benzyl-N-ethoxycarbonylamino)propylthio)-2-pyridyl)methylthio-1H-benzimidazole

Example 43

2-(3-methyl-4-(3-(N-benzyl-N-(2-ethoxycarbonylethyl)amino)propylthio)-2-pyridyl)methylthio-1H-benzimidazole

Example 44

2-(3-methyl-4-(3-(N-benzyl-N-(3-ethoxycarbonylpropyl)amino)propylthio)-2-pyridyl)methylthio-1H-benzimidazole

Example 45

2-(3-methyl-4-(3-(N-benzyl-N-(4-ethoxycarbonylbutyl)amino)propylthio)-2-pyridyl)methylthio-1H-benzimidazole

Example 46

2-(3-methyl-4-(3-(N-benzyl-N-(5-ethoxycarbonylpentyl)amino)propylthio)-2-pyridyl)methylthio-1H-benzimidazole

Example 47

2-(3-methyl-4-(3-(N-benzyl-N-(2-hydroxyethyl)amino)propylthio)-2-pyridyl)methylthio-1H-benzimidazole

Example 48

2-(3-methyl-4-(3-(N-benzyl-N-(3-hydroxypropyl)amino)propylthio)-2-pyridyl)methylthio-1H-benzimidazole

Example 49

2-(3-methyl-4-(3-(N-benzyl-N-(4-hydroxybutyl)amino)propylthio)-2-pyridyl)methylthio-1H-benzimidazole

Example 50

2-(3-methyl-4-(3-(N-benzyl-N-(2,3-dihydroxypropyl)amino)propylthio)-2-pyridyl)methylthio-1H-benzimidazole

Example 51

2-(3-Methyl-4-(2-aminoethylthio)-2-pyridyl)methylthio-1H-benzimidazole (2.0 g) was dissolved in dimethylformamide (20 ml) and thereto were added ethylene bromohydrin (0.83 g) and potassium carbonate (1.25 g). The mixture was stirred at 60-70 °C for 2 hours. The reaction mixture was poured into water and extracted with ethyl acetate. The extract was washed with water, dried over anhydrous magnesium sulfate and the solvent was distilled away under reduced pressure. The residue was subjected to column chromatography and eluted with chloroform-methanol. Ethanol-hydrochloric acid was added thereto. Recrystallization from ethanol-methanol gave 2-(3-methyl-4-(2-(2-hydroxyethylamino)ethylthio)-2-pyridyl) methylthio-1H-benzimidazole trihydrochloride hydrate as colorless crystals, melting point 215-217 °C (dec.).

The compounds of Examples 52-54 can be synthesized using 2-(3-methyl-4-(2-aminoethylthio)-2-pyridyl)methylthio-1H-benzimidazole as a starting compound.

Example 52

2-(3-methyl-4-(2-(3-hydroxypropylamino)ethylthio)-2-pyridyl)methylthio-1H-benzimidazole

Example 53

2-(3-methyl-4-(2-(ethoxycarbonylmethylamino)ethylthio)-2-pyridyl)methylthio-1H-benzimidazole dihydrochloride, melting point 233-234 °C (dec.)

Example 54

2-(3-methyl-4-(2-(ethoxycarbonylamino)ethylthio)-2-pyridyl)methylthio-1H-benzimidazole

Example 55

2-(3-Methyl-4-(3-aminopropylthio)-2-pyridyl)methylthio-1H-benzimidazole (2.0 g) was dissolved in dimethylformamide (20 ml) and thereto were added ethylene bromohydrin (0.83 g) and potassium carbonate (1.25 g). The mixture was stirred at 60-70 °C for 2 hours. The reaction mixture was poured into water and extracted with ethyl acetate. The extract was dried over anhydrous magnesium sulfate and the solvent was distilled away under reduced pressure. The residue was subjected to column chromatography and extracted with chloroform-methanol to give 2-(3-methyl-4-(3-(2-hydroxyethylamino)propylthio)-2-pyridyl) methylthio-1H-benzimidazole.

The compounds of Examples 56-58 can be synthesized using 2-(3-methyl-4-(3-aminopropylthio)-2-pyridyl)methylthio-1H-benzimidazole as a starting compound.

Example 56

2-(3-methyl-4-(3-(3-hydroxypropylamino)propylthio)-2-pyridyl)methylthio-1H-benzimidazole

Example 57

2-(3-methyl-4-(3-(2,3-dihydroxypropylamino)propylthio)-2-pyridyl)methylthio-1H-benzimidazole

Example 58

2-(3-methyl-4-(3-(ethoxycarbonylmethylamino)propylthio)-2-pyridyl)methylthio-1H-benzimidazole

Example 59

2-(3-Methyl-4-(2-aminoethylthio)-2-pyridyl)methylthio-1H-benzimidazole (2.0 g) was dissolved in dimethylformamide (20 ml) and thereto were added ethylene bromohydrin (1.59 g) and potassium carbonate (2.1 g). The mixture was stirred at 70-80 °C for 3 hours. The reaction mixture was poured into water and extracted with ethyl acetate. The extract was washed with water, dried over anhydrous magnesium sulfate and the solvent was distilled away under reduced pressure. The residue was subjected to column chromatography and eluted with chloroform-methanol to give 2-(3-methyl-4-(2-(N,N-bis(2-hydroxyethyl)-amino)ethylthio)-2-pyridyl)methylthio-1H-benzimidazole as a pale yellow oil.

The compounds of Examples 60-65 can be synthesized using 2-(3-methyl-4-(2-aminoethylthio)-2-pyridyl)methylthio-1H-benzimidazole as a starting compound.

Example 60

2-(3-methyl-4-(2-(N,N-bis(ethoxycarbonylmethyl)amino)ethylthio)-2-pyridyl)methylthio-1H-benzimidazole

Example 61

2-(3-methyl-4-(2-(N,N-bis(3-hydroxypropyl)amino)ethylthio)-2-pyridyl)methylthio-1H-benzimidazole

Example 62

2-(3-methyl-4-(2-(N,N-bis(2-ethoxycarbonylethyl)amino)ethylthio)-2-pyridyl)methylthio-1H-benzimidazole

Example 63

2-(3-methyl-4-(2-(N,N-bis(3-ethoxycarbonylpropyl)amino)ethylthio)-2-pyridyl)methylthio-1H-benzimidazole

Example 64

2-(3-methyl-4-(2-(N,N-bis(4-ethoxycarbonylbutyl)amino)ethylthio)-2-pyridyl)methylthio-1H-benzimidazole

Example 65

2-(3-methyl-4-(2-(N,N-diethoxycarbonylamino)ethylthio)-2-pyridyl)methylthio-1H-benzimidazole

Example 66

2-(3-Methyl-4-(3-aminopropylthio)-2-pyridyl)methylthio-1H-benzimidazole (2.0 g) was dissolved in dimethylformamide (20 ml) and thereto were added ethylene bromohydrin (1.52 g) and potassium carbonate (2.0 g). The mixture was stirred at 70-80 °C for 3 hours. The reaction mixture was poured into water and extracted with ethyl acetate. The extract was washed with water, dried over anhydrous magnesium sulfate and the solvent was distilled away under reduced pressure. The residue was subjected to column chromatography and eluted with chloroform-methanol to give 2-(3-methyl-4-(3-(N,N-bis(2-hydroxyethyl)-amino)propylthio)-2-pyridyl)methylthio-1H-benzimidazole as a pale yellow oil.

The compounds of Examples 67-71 can be synthesized using 2-(3-methyl-4-(3-aminopropylthio)-2-pyridyl)methylthio-1H-benzimidazole as a starting compound.

Example 67

2-(3-methyl-4-(3-(N,N-bis(ethoxycarbonylmethyl)amino)propylthio)-2-pyridyl)methylthio-1H-benzimidazole

Example 68

2-(3-methyl-4-(3-(N,N-bis(3-hydroxypropyl)amino)propylthio)-2-pyridyl)methylthio-1H-benzimidazole

Example 69

2-(3-methyl-4-(3-(N,N-bis(2-ethoxycarbonylethyl)amino)propylthio)-2-pyridyl)methylthio-1H-benzimidazole

Example 70

2-(3-methyl-4-(3-(N,N-bis(3-ethoxycarbonylpropyl)amino)propylthio)-2-pyridyl)methylthio-1H-benzimidazole

Example 71

2-(3-methyl-4-(3-(N,N-bis(4-ethoxycarbonylbutyryl)amino)propylthio)-2-pyridyl)methylthio-1H-benzimidazole

Example 72

2-(3-methyl-4-(3-(N,N-diethoxycarbonylamino)propylthio)-2-pyridyl)methylthio-1H-benzimidazole

Example 73

2-(3-Methyl-4-(2-(N-benzyl-N-ethoxycarbonylmethylamino)ethylthio)-2-pyridyl)methylthio-1H-benzimidazole (1.0 g) was dissolved in ethanol (10 ml) and thereto was added an aqueous solution (3 ml) of 1N sodium hydroxide. The mixture was stirred at room temperature for 1 hour. The reaction mixture was concentrated under reduced pressure and adjusted to pH 4 with an aqueous solution of dilute acetic acid. The resultant crystals were collected by filtration to give 2-(3-methyl-4-(2-(N-benzyl-N-carboxymethylamino)ethylthio)-2-pyridyl)methylthio-1H-benzimidazole as colorless crystals.

The compounds of Examples 74-95 can be synthesized in the similar manner.

Example 74

2-(3-methyl-4-(2-(N-benzyl-N-(2-carboxyethyl)amino)ethylthio)-2-pyridyl)methylthio-1H-benzimidazole

Example 75

2-(3-methyl-4-(2-(N-benzyl-N-(3-carboxypropyl)amino)ethylthio)-2-pyridyl)methylthio-1H-benzimidazole

Example 76

2-(3-methyl-4-(2-(N-benzyl-N-(4-carboxybutyl)amino)ethylthio)-2-pyridyl)methylthio-1H-benzimidazole

Example 77

2-(3-methyl-4-(2-(N-benzyl-N-carboxyamino)ethylthio)-2-pyridyl)methylthio-1H-benzimidazole

Example 78

2-(3-methyl-4-(3-(N-benzyl-N-carboxymethylamino)propylthio)-2-pyridyl)methylthio-1H-benzimidazole

Example 79

2-(3-methyl-4-(3-(N-benzyl-N-(2-carboxyethyl)amino)propylthio)-2-pyridyl)methylthio-1H-benzimidazole

Example 80

2-(3-methyl-4-(3-(N-benzyl-N-(3-carboxypropyl)amino)propylthio)-2-pyridyl)methylthio-1H-benzimidazole

Example 81

2-(3-methyl-4-(3-(N-benzyl-N-(4-carboxybutyl)amino)propylthio)-2-pyridyl)methylthio-1H-benzimidazole

Example 82

2-(3-methyl-4-(2-(carboxymethylamino)ethylthio)-2-pyridyl)methylthio-1H-benzimidazole, melting point 215-216°C (dec.)

Example 83

2-(3-methyl-4-(2-(2-carboxyethylamino)ethylthio)-2-pyridyl)methylthio-1H-benzimidazole

Example 84

2-(3-methyl-4-(2-(3-carboxypropylamino)ethylthio)-2-pyridyl)methylthio-1H-benzimidazole

Example 85

2-(3-methyl-4-(2-(4-carboxybutylamino)ethylthio)-2-pyridyl)methylthio-1H-benzimidazole

Example 86

2-(3-methyl-4-(2-(N,N-dicarboxymethylamino)ethylthio)-2-pyridyl)methylthio-1H-benzimidazole

Example 87

2-(3-methyl-4-(2-(N,N-bis(2-carboxyethyl)amino)ethylthio)2-pyridyl)methylthio-1H-benzimidazole

Example 88

2-(3-methyl-4-(2-(N,N-bis(3-carboxypropyl)amino)ethylthio)2-pyridyl)methylthio-1H-benzimidazole

Example 89

2-(3-methyl-4-(2-(N,N-bis(4-carboxybutyl)amino)ethylthio)2-pyridyl)methylthio-1H-benzimidazole

Example 90

2-(3-methyl-4-(2-dimethylamino-2-carboxyethyl)thio-2-pyridyl)methylthio-1H-benzimidazole

Example 91

2-(3-methyl-4-(2-amino-2-carboxyethylthio)-2-pyridyl)methylthio-1H-benzimidazole

Example 92

2-(3-methyl-4-(2-morpholino-2-carboxyethylthio)-2-pyridyl)methylthio-1H-benzimidazole

Example 93

2-(3-methyl-4-(2-(1,2,3,4-tetrahydroisoquinolin-2-yl)-2-carboxyethylthio)-2-pyridyl)methylthio-1H-benzimidazole

Example 94

2-(3-methyl-4-(2-(2-carboxymethoxymethyl)morpholinoethylthio)-2-pyridyl)methylthio-1H-benzimidazole

Example 95

2-(3-methyl-4-(2-(2-carboxymethylaminomethylmorpholino)ethylthio)-2-pyridyl)methylthio-1H-benzimidazole

Example 96

2-Mercaptobenzimidazole (1.5 g) was dissolved in ethanol (20 ml) and thereto were added ethyl 2-dimethylamino-3-(2-chloromethyl-3-methyl-4-pyridylthio)propionate (3.16 g) and an aqueous solution (10 ml) of 1N sodium hydroxide. The mixture was stirred at 50 °C for 1 hour. The reaction mixture was concentrated under reduced pressure and water was added to the residue. The mixture was extracted with ethyl acetate. The extract was washed with water, dried over anhydrous magnesium sulfate and the solvent was distilled away under reduced pressure. The residue was subjected to column chromatography and eluted with chloroform-methanol to give 2-(3-methyl-4-(2-dimethylamino-2-ethoxycarbonylethylthio)-2-pyridyl)methylthio-1H-benzimidazole as a pale yellow oil.

The compounds of Examples 97-101 can be synthesized in the similar manner.

Example 97

2-(3-methyl-4-(2-amino-2-ethoxycarbonylethylthio)-2-pyridyl)methylthio-1H-benzimidazole

Example 98

2-(3-methyl-4-(2-morpholino-2-ethoxycarbonylethylthio)-2-pyridyl)methylthio-1H-benzimidazole

Example 99

2-(3-methyl-4-(2-(1,2,3,4-tetrahydroisoquinolin-2-yl)-2-ethoxycarbonylethylthio)-2-pyridyl)methylthio-1H-benzimidazole

Example 100

2-(3-methyl-4-(2-(2-ethoxycarbonylmethoxymethylmorpholino)ethylthio)-2-pyridyl)methylthio-1H-benzimidazole

Example 101

2-(3-methyl-4-(2-(2-ethoxycarbonylmethylaminomethylmorpholino)ethythio)-2-pyridyl))methylthio-1H-benzimidazole

Example 102

2-(3-Methyl-4-(2-(2-aminoethylthio)-2-pyridyl)methylthio-1H-benzimidazole (2.0 g) was dissolved in ethanol (20 ml) and thereto was added phenyl isocyanate (0.79 ml). The mixture was stirred for 5.5 hours. The ethanol was evaporated under reduced pressure and the resultant crystals were collected by filtration to give N-phenyl-N'-(2-(2-(1H-benzimidazol-2-yl)thiomethyl)-3-methyl-4-pyridylthio)ethylurea, melting point 208-210 °C.

The compounds as shown in the following Tables were produced in the similar manner. In Tables, each symbol means the following.

Me : methyl Et : ethyl i-Pr : isopropyl

Bu : butyl Ac : acetyl

Table 4

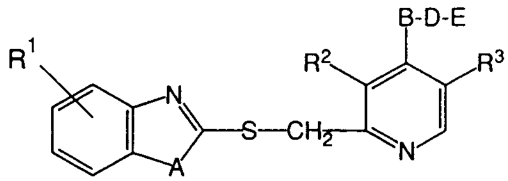

| No | $R^1$ | $R^2$ | $R^3$ | A | B | D | E |
|---|---|---|---|---|---|---|---|
| 201 | 5-Cl | $CH_3$ | H | NH | S | $CH_2CH_2$ | 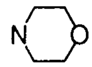 |
| 202 | 5-Me | $CH_3$ | H | NH | S | $CH_2CH_2$ | 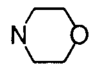 |
| 203 | 5-MeO | $CH_3$ | H | NH | S | $CH_2CH_2$ | 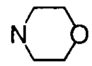 |
| 204 | 5-OH | $CH_3$ | H | NH | S | $CH_2CH_2$ | 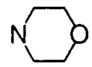 |
| 205 | 5-COOEt | $CH_3$ | H | NH | S | $CH_2CH_2$ | 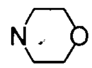 |
| 206 | 5-$CF_3$ | $CH_3$ | H | NH | S | $CH_2CH_2$ | 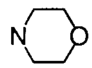 |
| 207 | 5-$NO_2$ | $CH_3$ | H | NH | S | $CH_2CH_2$ | 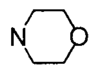 |
| 208 | 5-$NH_2$ | $CH_3$ | H | NH | S | $CH_2CH_2$ | 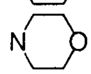 |
| 209 | 5-NHMe | $CH_3$ | H | NH | S | $CH_2CH_2$ | 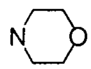 |
| 210 | 5-$NMe_2$ | $CH_3$ | H | NH | S | $CH_2CH_2$ | 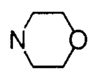 |
| 211 | 5$NHCH_2COOH$ | $CH_3$ | H | NH | S | $CH_2CH_2$ | 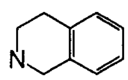 |
| 212 | 5$NHCH_2COOEt$ | $CH_3$ | H | NH | S | $CH_2CH_2$ | 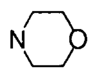 |

Table 4 (continued)

| No | $R^1$ | $R^2$ | $R^3$ | A | B | D | E |
|---|---|---|---|---|---|---|---|
| 213 | $5NHCH_2COOH$ | $CH_3$ | H | NH | S | $CH_2CH_2$ | 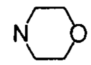 |
| 214 | $5\text{-}NEt_2$ | $CH_3$ | H | NH | S | $CH_2CH_2$ | 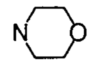 |
| 215 | 5-COOH | $CH_3$ | H | NH | NH | — | 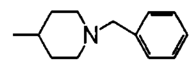 |
| 216 | H | $CH_3$ | $CH_3$ | $NCH_2CH_2OH$ | O | — | 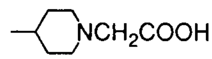 |
| 217 | H | $CH_3$ | H | S | S | $CH_2CH_2$ | 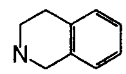 |
| 218 | H | $CH_3$ | H | O | S | $CH_2CH_2$ | 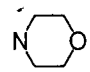 |
| 219 | H | $CH_3$ | H | O | S | $CH_2CH_2$ | 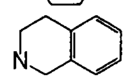 |
| 220 | H | $CH_3$ | H | $NCONH_2$ | O | $CH_2CH_2$ | 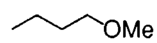 |
| 221 | H | $CH_3$ | H | S | NH | $CH_2CH_2$ | 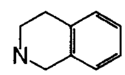 |
| 222 | H | $CH_3$ | H | S | NH | $CH_2CH_2$ | 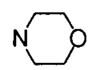 |
| 223 | $5NH(CH_2)_2COOH$ | $CH_3$ | H | NH | S | $CH_2CH_2$ | 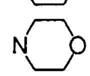 |
| 224 | $5NH(CH_2)_3COOH$ | $CH_3$ | H | NH | S | $CH_2CH_2$ | 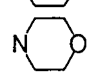 |
| 225 | H | $CH_3$ | H | NH | S | $CH_2CH_2$ | 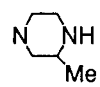 |

Table 5

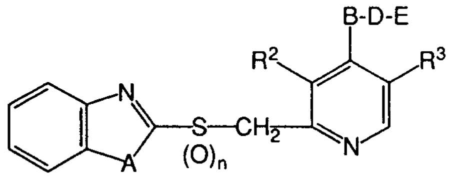

| No | n | $R^2$ | $R^3$ | A | B | D | E |
|---|---|---|---|---|---|---|---|
| 301 | 1 | $CH_3$ | H | NH | S | $CH_2CH_2$ | 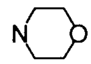 |
| 302 | 1 | $CH_3$ | H | NH | S | $CH_2CH_2$ | 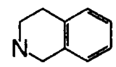 |
| 303 | 1 | $CH_3$ | H | NH | S | $CH_2CH_2$ | 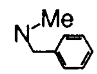 |
| 304 | 1 | $CH_3$ | H | NH | S | $CH_2CH_2$ | 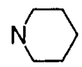 |
| 305 | 1 | $CH_3$ | H | NH | S | $CH_2CH_2$ | 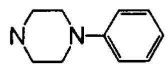 |
| 306 | 1 | $CH_3$ | H | NH | S | $CH_2CH_2$ | 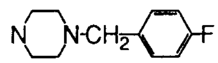 |
| 307 | 1 | $CH_3$ | H | NH | S | $CH_2CH_2$ | 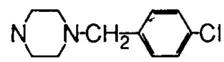 |
| 308 | 2 | $CH_3$ | H | NH | S | $CH_2CH_2$ | 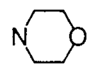 |
| 309 | 2 | $CH_3$ | H | NH | S | $CH_2CH_2$ | 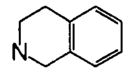 |
| 310 | 1 | $CH_3$ | H | NH | $SO_2$ | $CH_2CH_2$ | 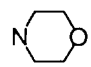 |
| 311 | 1 | $CH_3$ | H | NH | $SO_2$ | $CH_2CH_2$ | 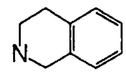 |
| 312 | 1 | $CH_3$ | H | NH | $SO_2$ | $CH_2CH_2$ | 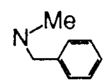 |
| 313 | 1 | $CH_3$ | H | NH | $SO_2$ | $CH_2CH_2$ | 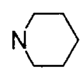 |
| 314 | 1 | $CH_3$ | H | NH | $SO_2$ | $CH_2CH_2$ | 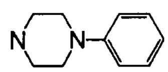 |
| 315 | 1 | $CH_3$ | H | NCOOMe | $SO_2$ | — | 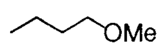 |
| 316 | 1 | $CH_3$ | H | NH | S | — | 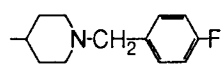 |
| 317 | 1 | $CH_3$ | H | NH | S | — | 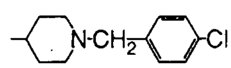 |

Table 5 (continued)

| No | n | $R^2$ | $R^3$ | A | B | D | E |
|---|---|---|---|---|---|---|---|
| 318 | 1 | $CH_3$ | H | $NCONH_2$ | O | $CH_2CH_2$ | 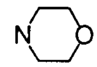 |
| 319 | 1 | $CH_3$ | H | $NCONH_2$ | O | $CH_2CH_2$ | 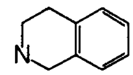 |
| 320 | 1 | $CH_3$ | H | $NCONH_2$ | O | $CH_2CH_2$ | 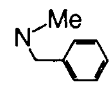 |
| 321 | 1 | $CH_3$ | H | $NCONH_2$ | O | $CH_2CH_2$ | 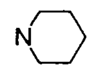 |
| 322 | 1 | $CH_3$ | H | $NCH_2CH_2OH$ | O | $CH_2CH_2$ | 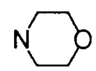 |
| 323 | 1 | $CH_3$ | H | $NCH_2CH_2OH$ | O | $CH_2CH_2$ | 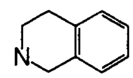 |
| 324 | 1 | $CH_3$ | H | $NCH_2CH_2OH$ | O | $CH_2CH_2$ | 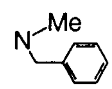 |
| 325 | 1 | $CH_3$ | H | $NCH_2CH_2OH$ | O | $CH_2CH_2$ | 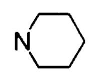 |
| 326 | 1 | $CH_3$ | H | $NCH_2COOH$ | O | $CH_2CH_2$ | 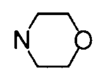 |
| 327 | 1 | $CH_3$ | H | $NCH_2COOH$ | O | $CH_2CH_2$ | 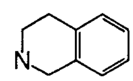 |
| 328 | 1 | $CH_3$ | H | $NCH_2COOH$ | O | $CH_2CH_2$ | 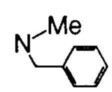 |
| 329 | 1 | $CH_3$ | H | $NCH_2COOH$ | O | $CH_2CH_2$ | 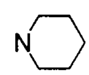 |
| 330 | 1 | $CH_3$ | H | NCONHMe | O | — | 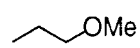 |
| 331 | 1 | $CH_3$ | H | NCONHMe | O | $(CH_2)_3$ | NH-Bu |
| 332 | 1 | $CH_3$ | H | $NCH_3$ | NH | — | 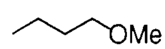 |
| 333 | 1 | $CH_3$ | H | $NCH_2CH_2OMe$ | O | $CH_2CH_2$ | 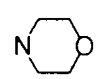 |
| 334 | 1 | $CH_3$ | H | $NCH_2CH_2OAc$ | O | $CH_2CH_2$ | 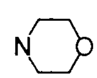 |

Table 6

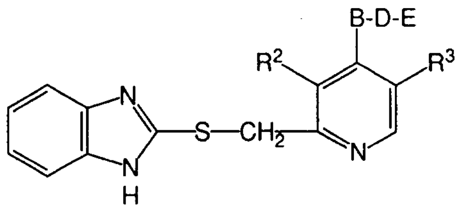

| No | $R^2$ | $R^3$ | B | D | E |
|---|---|---|---|---|---|
| 401 | $CH_3$ | H | S | $CH_2CH_2$ | 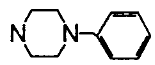 |
| 402 | $CH_3$ | H | S | — | 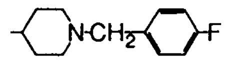 |
| 403 | $CH_3$ | H | S | — | 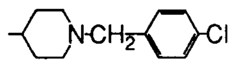 |
| 404 | $CH_3$ | H | NH | $CH_2CH_2$ | 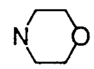 |
| 405 | $CH_3$ | H | NMe | $CH_2CH_2$ | 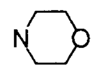 |
| 406 | $CH_3$ | H | NH | $CH_2CH_2$ | 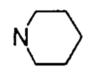 |
| 407 | $CH_3$ | H | NMe | $CH_2CH_2$ | 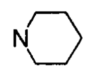 |
| 408 | $CH_3$ | H | S | $CH_2CH_2$ | 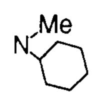 |
| 409 | $CH_3$ | H | S | $CH_2CH_2$ | 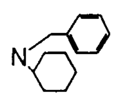 |
| 410 | $CH_3$ | H | S | $CH_2CH_2$ | 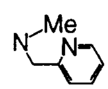 |
| 411 | $CH_3$ | H | S | $CH_2CH_2$ | 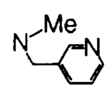 |
| 412 | $CH_3$ | H | S | $CH_2CH_2$ | 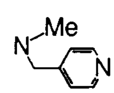 |
| 413 | $CH_3$ | H | S | $(CH_2)_4$ | 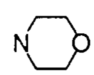 |
| 414 | $CH_3$ | H | S | $(CH_2)_5$ | 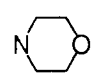 |
| 415 | $CH_3$ | H | S | $(CH_2)_3$ | 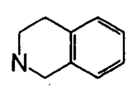 |

Table 6 (continued)

| No | $R^2$ | $R^3$ | B | D | E |
|---|---|---|---|---|---|
| 416 | $CH_3$ | H | S | $(CH_2)_4$ | 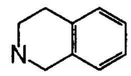 |
| 417 | $CH_3$ | H | S | $(CH_2)_5$ | 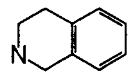 |
| 418 | $CH_3$ | H | S | $CH_2CH_2$ | 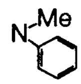 |
| 419 | $CH_3$ | H | S | $CH_2CH_2$ | 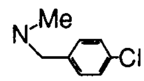 |
| 420 | $CH_3$ | H | S | $CH_2CH_2$ | 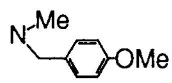 |
| 421 | $CH_3$ | H | S | $CH_2CH_2$ | 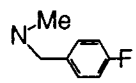 |
| 422 | $CH_3$ | H | S | $CH_2CH_2$ | 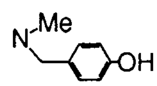 |
| 423 | $CH_3$ | H | S | $CH_2CH_2$ | 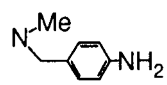 |
| 424 | $CH_3$ | H | S | $(CH_2)_3$ | 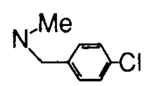 |
| 425 | $CH_3$ | H | S | $(CH_2)_3$ | 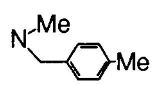 |
| 426 | $CH_3$ | H | S | $(CH_2)_3$ | 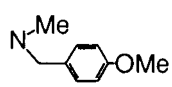 |
| 427 | $CH_3$ | H | S | $(CH_2)_3$ | 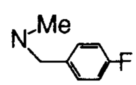 |
| 428 | $CH_3$ | H | S | $(CH_2)_3$ | 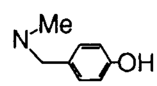 |
| 429 | $CH_3$ | H | S | $(CH_2)_3$ | 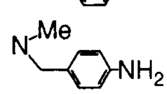 |
| 430 | $CH_3$ | H | S | — | 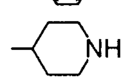 |

Table 6 (continued)

| No | $R^2$ | $R^3$ | B | D | E |
|---|---|---|---|---|---|
| 431 | $CH_3$ | H | S | — | 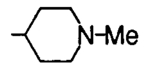 |
| 432 | $CH_3$ | H | S | — | 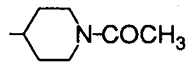 |
| 433 | $CH_3$ | H | S | $CH_2$ | 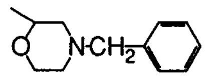 |
| 434 | $CH_3$ | H | S | $CH_2$ | 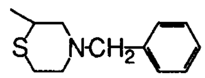 |
| 435 | $CH_3$ | H | S | $CH_2CH_2$ | 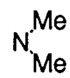 |
| 436 | $CH_3$ | H | S | $(CH_2)_3$ | 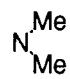 |
| 437 | $CH_3$ | H | S | $CH_2CH_2$ | $NH_2$ |
| 438 | $CH_3$ | H | S | $(CH_2)_3$ | $NH_2$ |
| 439 | $CH_3$ | H | S | $CH_2CH_2$ | 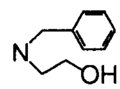 |
| 440 | $CH_3$ | H | S | $CH_2CH_2$ | 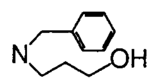 |
| 441 | $CH_3$ | H | S | $CH_2CH_2$ | 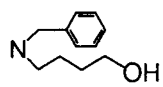 |
| 442 | $CH_3$ | H | S | $(CH_2)_3$ | 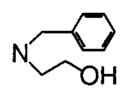 |
| 443 | $CH_3$ | H | S | $(CH_2)_3$ | 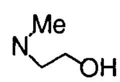 |
| 444 | $CH_3$ | H | S | $(CH_2)_3$ | 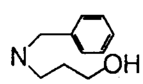 |
| 445 | $CH_3$ | H | S | $(CH_2)_4$ | 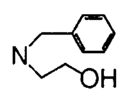 |

Table 6 (continued)

| No | $R^2$ | $R^3$ | B | D | E |
|---|---|---|---|---|---|
| 446 | $CH_3$ | H | S | $(CH_2)_4$ | 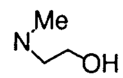 |
| 447 | $CH_3$ | H | S | $(CH_2)_4$ | 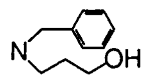 |
| 448 | $CH_3$ | H | S | $CH_2CH_2$ | 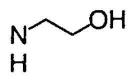 |
| 449 | $CH_3$ | H | S | $(CH_2)_3$ | 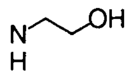 |
| 450 | $CH_3$ | H | S | $CH_2CH_2$ | 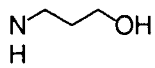 |
| 451 | $CH_3$ | H | S | $(CH_2)_3$ | 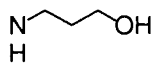 |
| 452 | $CH_3$ | H | S | $CH_2CH_2$ | 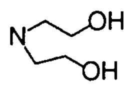 |
| 453 | $CH_3$ | H | S | $(CH_2)_3$ | 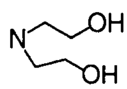 |
| 454 | $CH_3$ | H | S | $CH_2CH_2$ | 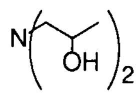 |
| 455 | $CH_3$ | H | S | $CH_2CH_2$ | 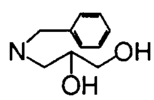 |
| 456 | $CH_3$ | H | S | $(CH_2)_3$ | 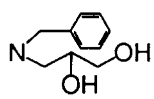 |
| 457 | $CH_3$ | H | S | $(CH_2)_3$ | 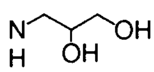 |
| 458 | $CH_3$ | H | S | $CH_2CH_2$ | 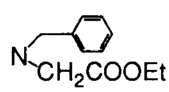 |
| 459 | $CH_3$ | H | S | $CH_2CH_2$ | 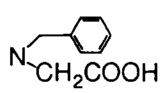 |
| 460 | $CH_3$ | H | S | $CH_2CH_2$ | 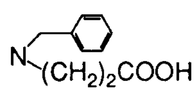 |

Table 6 (continued)

| No | $R^2$ | $R^3$ | B | D | E |
|---|---|---|---|---|---|
| 461 | $CH_3$ | H | S | $CH_2CH_2$ | 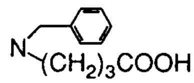 |
| 462 | $CH_3$ | H | S | $(CH_2)_3$ | 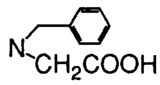 |
| 463 | $CH_3$ | H | S | $(CH_2)_3$ | 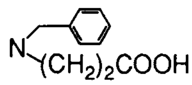 |
| 464 | $CH_3$ | H | S | $CH_2CH_2$ | NHCSNH-Me |
| 465 | $CH_3$ | H | S | $CH_2CH_2$ | NHCSNH-Et |
| 466 | $CH_3$ | H | S | $CH_2CH_2$ | NHCSNH-Bu |
| 467 | $CH_3$ | H | S | $CH_2CH_2$ | NHCSNH- 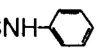 |
| 468 | $CH_3$ | H | S | $CH_2CH_2$ | NHCONH-Me |
| 469 | $CH_3$ | H | S | $CH_2CH_2$ | NHCONH-Et |
| 470 | $CH_3$ | H | S | $CH_2CH_2$ | NHCONH-Bu |
| 471 | $CH_3$ | H | S | $CH_2CH_2$ | NHCONH- 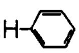 |
| 472 | $CH_3$ | H | S | $(CH_2)_3$ | NHCSNH-Bu |
| 473 | $CH_3$ | H | S | $(CH_2)_3$ | NHCSNH- 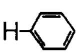 |
| 474 | $CH_3$ | H | S | $(CH_2)_3$ | NHCONH-Bu |
| 475 | $CH_3$ | H | S | $(CH_2)_3$ | NHCONH- 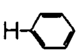 |

Table 6 (continued)

| No | $R^2$ | $R^3$ | B | D | E |
|---|---|---|---|---|---|
| 476 | $CH_3$ | H | S | $CH_2CH_2$ | 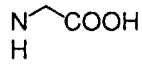 |
| 477 | $CH_3$ | H | S | $(CH_2)_3$ | 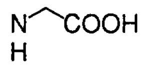 |
| 478 | $CH_3$ | H | S | $CH_2CH_2$ | 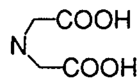 |
| 479 | $CH_3$ | H | S | $CH_2CH_2$ | 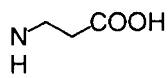 |
| 480 | $CH_3$ | H | S | $CH_2CH_2$ | 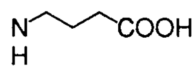 |
| 481 | $CH_3$ | H | S | 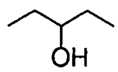 | $NH_2$ |
| 482 | $CH_3$ | H | S | 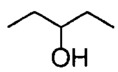 | NH-Et |
| 483 | $CH_3$ | H | S | 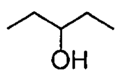 | $N(i\text{-}Pr)_2$ |
| 484 | $CH_3$ | H | S | $CH_2CH_2$ | 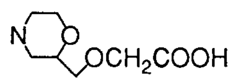 |
| 485 | $CH_3$ | H | S | $(CH_2)_3$ | 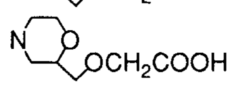 |
| 486 | $CH_3$ | H | S | $CH_2CH_2$ | 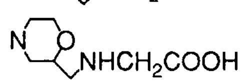 |
| 487 | $CH_3$ | H | S | $(CH_2)_3$ | 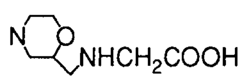 |
| 488 | $CH_3$ | H | S | $(CH_2)_3$ | 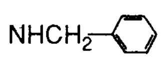 |
| 489 | $CH_3$ | H | S | 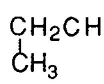 | 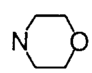 |
| 490 | $CH_3$ | H | S | 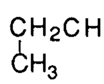 | 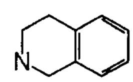 |

Table 6 (continued)

| No | $R^2$ | $R^3$ | B | D | E |
|---|---|---|---|---|---|
| 491 | $CH_3$ | H | S | $CH_2CH_2$ | NH-Me |
| 492 | $CH_3$ | H | S | $CH_2CH_2$ | NH-Bu |
| 493 | $CH_3$ | H | S | $CH_2CH$<br>$COOH$ | $NH_2$ |
| 494 | $CH_3$ | H | S | $CH_2CH$<br>$COOH$ | 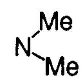 |
| 495 | $CH_3$ | H | S | $CH_2CH$<br>$COOH$ | 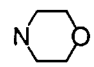 |
| 496 | $CH_3$ | H | S | $CH_2CH$<br>$COOH$ | 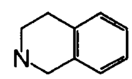 |
| 497 | $CH_3$ | H | S | $CH_2CH$<br>$COOEt$ | $NH_2$ |
| 498 | $CH_3$ | H | S | $CH_2CH$<br>$COOEt$ | 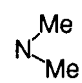 |
| 499 | $CH_3$ | H | S | $CH_2CH$<br>$CONHMe$ | $NH_2$ |
| 500 | $CH_3$ | H | S | $CH_2CH$<br>$CONHEt$ | 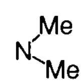 |
| 501 | $CH_3$ | H | S | $CH_2CH_2$ | 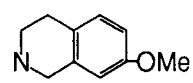 |
| 502 | $CH_3$ | H | S | $CH_2CH_2$ | 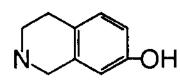 |
| 503 | $CH_3$ | H | S | $CH_2CH_2$ | 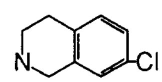 |
| 504 | $CH_3$ | H | S | $CH_2CH_2$ | 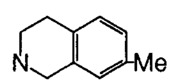 |
| 505 | $CH_3$ | H | S | $CH_2CH_2$ | 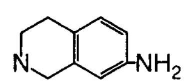 |

Table 6 (continued)

| No | $R^2$ | $R^3$ | B | D | E |
|---|---|---|---|---|---|
| 506 | $CH_3$ | H | S | $CH_2CH_2$ | 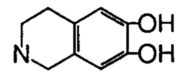 |
| 507 | $CH_3$ | H | S | $CH_2CH_2$ | 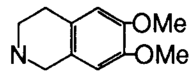 |
| 508 | $CH_3$ | H | S | $CH_2CH_2$ | 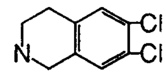 |
| 509 | $CH_3$ | H | S | $CH_2CH_2$ | 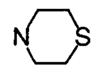 |
| 510 | $CH_3$ | H | S | $CH_2CH_2$ | 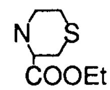 |
| 511 | $CH_3$ | H | S | $CH_2CH_2$ | 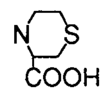 |
| 512 | $CH_3$ | H | S | $CH_2CH_2$ | 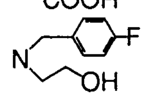 |
| 513 | $CH_3$ | H | S | $CH_2CH_2$ | 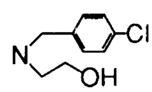 |
| 514 | $CH_3$ | H | S | $CH_2CH_2$ | 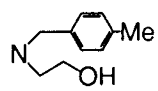 |
| 515 | $CH_3$ | H | S | $CH_2CH_2$ | 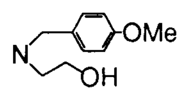 |
| 516 | $CH_3$ | H | S | $CH_2CH_2$ | 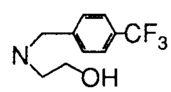 |
| 517 | $CH_3$ | H | S | $CH_2CH_2$ | 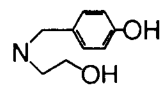 |
| 518 | $CH_3$ | H | S | $CH_2CH_2$ | 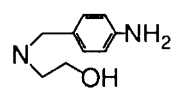 |
| 519 | $CH_3$ | H | S | $CH_2CH_2$ | 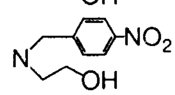 |
| 520 | $CH_3$ | H | S | $(CH_2)_3$ | 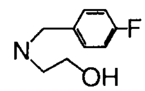 |

Table 6 (continued)

| No | $R^2$ | $R^3$ | B | D | E |
|---|---|---|---|---|---|
| 521 | $CH_3$ | H | S | $CH_2CH_2$ | 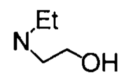 |
| 522 | $CH_3$ | H | S | $CH_2CH_2$ | 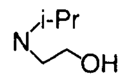 |
| 523 | $CH_3$ | H | S | $CH_2CH_2$ | 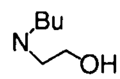 |
| 524 | $CH_3$ | H | S | $CH_2CH_2$ | 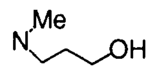 |
| 525 | $CH_3$ | H | S | $CH_2CH_2$ | 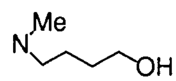 |
| 526 | $CH_3$ | H | S | $CH_2CH_2$ | 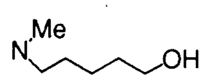 |
| 527 | $CH_3$ | H | S | $CH_2CH_2$ | 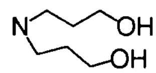 |
| 528 | $CH_3$ | H | S | $CH_2CH_2$ | 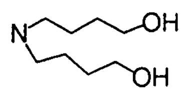 |
| 529 | $CH_3$ | H | S | $CH_2CH_2$ | 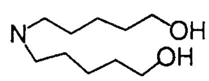 |
| 530 | $CH_3$ | H | S | $CH_2CH_2$ | 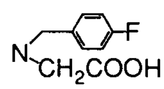 |
| 531 | $CH_3$ | H | S | $CH_2CH_2$ | 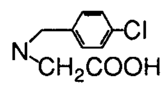 |
| 532 | $CH_3$ | H | S | $CH_2CH_2$ | 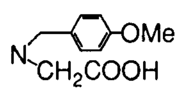 |
| 533 | $CH_3$ | H | S | $CH_2CH_2$ | 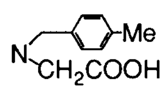 |
| 534 | $CH_3$ | H | S | $CH_2CH_2$ | 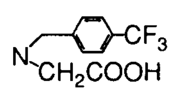 |
| 535 | $CH_3$ | H | S | $CH_2CH_2$ | 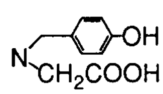 |

Table 6 (continued)

| No | $R^2$ | $R^3$ | B | D | E |
|---|---|---|---|---|---|
| 536 | $CH_3$ | H | S | $CH_2CH_2$ | 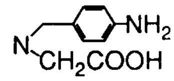 |
| 537 | $CH_3$ | H | S | $CH_2CH_2$ | 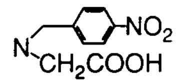 |
| 538 | $CH_3$ | H | S | $CH_2CH_2$ | 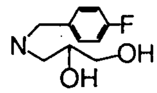 |
| 539 | $CH_3$ | H | S | $CH_2CH_2$ | 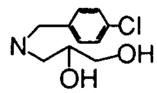 |
| 540 | $CH_3$ | H | S | $(CH_2)_3$ | 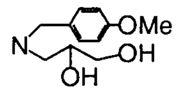 |
| 541 | $CH_3$ | H | S | $CH_2CH_2$ | 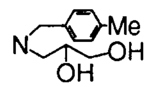 |
| 542 | $CH_3$ | H | S | $CH_2CH_2$ | 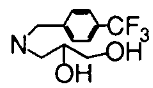 |
| 543 | $CH_3$ | H | S | $CH_2CH_2$ | 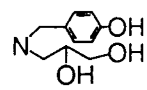 |
| 544 | $CH_3$ | H | S | $CH_2CH_2$ | 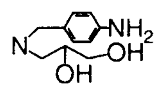 |
| 545 | $CH_3$ | H | S | $CH_2CH_2$ | 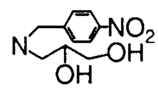 |
| 546 | $CH_3$ | H | S | $CH_2CH_2$ | $NHCONHCH_2$- 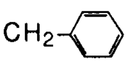 |
| 547 | $CH_3$ | H | S | $CH_2CH_2$ | $NHCONH(CH_2)_2$ - 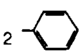 |
| 548 | $CH_3$ | H | S | $CH_2CH_2$ | $NHCSNHCH_2$ - 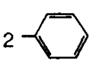 |
| 549 | $CH_3$ | H | S | $CH_2CH_2$ | $NHCSNH(CH_2)_2$ - 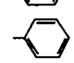 |
| 550 | $CH_3$ | H | S | $CH_2CH_2$ | 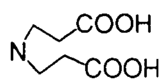 |

Table 6 (continued)

| No | $R^2$ | $R^3$ | B | D | E |
|---|---|---|---|---|---|
| 551 | $CH_3$ | H | S | $CH_2CH_2$ | 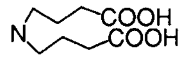 |
| 552 | $CH_3$ | H | S | $CH_2CH_2$ | 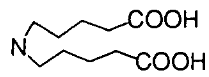 |
| 553 | $CH_3$ | H | S | $CH_2CH_2$ | 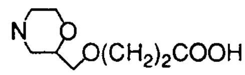 |
| 554 | $CH_3$ | H | S | $CH_2CH_2$ | 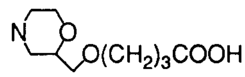 |
| 555 | $CH_3$ | H | S | $CH_2CH_2$ | 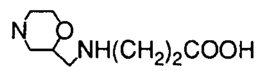 |
| 556 | $CH_3$ | H | S | $CH_2CH_2$ | 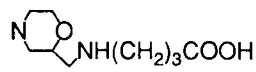 |
| 557 | $CH_3$ | H | S | $CH_2CH$<br>$COOH$ | NH-Me |
| 558 | $CH_3$ | H | S | $CH_2CH$<br>$COOH$ | $NHCH_2$- 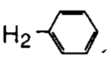 |
| 559 | $CH_3$ | H | S | $CH_2CH$<br>$COOH$ | 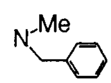 |
| 560 | $CH_3$ | H | S | $CH_2CH$<br>$COOH$ | 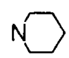 |
| 561 | $CH_3$ | H | S | $CH_2CH$<br>$COOH$ | 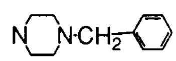 |
| 562 | $CH_3$ | H | S | $CH_2CH$<br>$COOH$ | 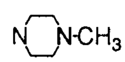 |
| 563 | $CH_3$ | H | S | $CH_2CH$<br>$COOH$ | 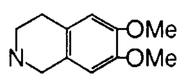 |
| 564 | $CH_3$ | H | S | $CH_2CH$<br>$COOEt$ | 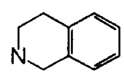 |
| 565 | $CH_3$ | H | S | $CH_2CH$<br>$COOEt$ | 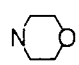 |
| 566 | $CH_3$ | H | S | $CH_2CH_2$ | NH–COOEt |
| 567 | $CH_3$ | H | S | $CH_2CH_2$ | 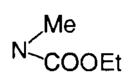 |
| 568 | $CH_3$ | H | S | $CH_2CH_2$ | 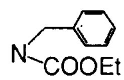 |

Table 7

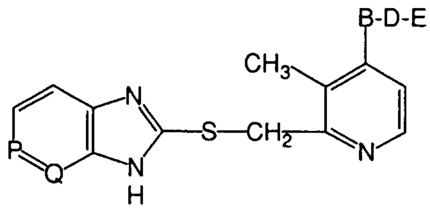

| No | P | Q | B | D | E |
|---|---|---|---|---|---|
| 601 | CH | N | S | $CH_2CH_2$ | 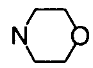 |
| 602 | N | CH | S | $CH_2CH_2$ | 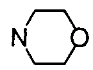 |
| 603 | CH | N | S | $CH_2CH_2$ | 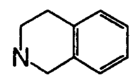 |
| 604 | N | CH | S | $CH_2CH_2$ | 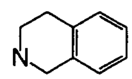 |
| 605 | CH | N | S | $(CH_2)_3$ | 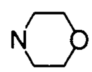 |
| 606 | CH | N | S | $(CH_2)_4$ | 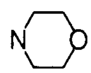 |
| 607 | CH | N | S | $CH_2CH_2$ | 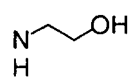 |
| 608 | N | CH | S | $CH_2CH_2$ | 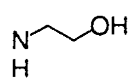 |
| 609 | CH | N | S | $CH_2CH_2$ | 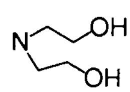 |
| 610 | N | CH | S | $CH_2CH_2$ | 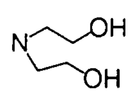 |

Table 7 (continued)

| No | P | Q | B | D | E |
|---|---|---|---|---|---|
| 611 | CH | N | S | $CH_2CH_2$ | 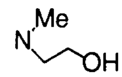 |
| 612 | N | CH | S | $CH_2CH_2$ | 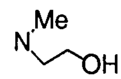 |
| 613 | CH | N | S | $CH_2CH_2$ | 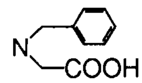 |
| 614 | N | CH | S | $CH_2CH_2$ | 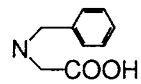 |
| 615 | CH | N | S | $CH_2CH_2$ | 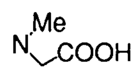 |
| 616 | N | CH | S | $CH_2CH_2$ | 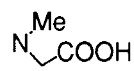 |
| 617 | CH | N | S | $CH_2CH_2$ | 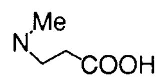 |
| 618 | N | CH | S | $CH_2CH_2$ | 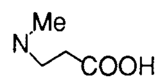 |
| 619 | CH | N | S | 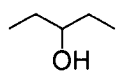 | NH-Et |
| 620 | N | CH | S | 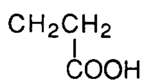 | $NH_2$ |

Formulation Examples

Tablets The tablets containing 20 mg of the active ingredient are prepared according to the following formulation.

| | |
|---|---|
| Compound (I) | 20 mg |
| Corn starch | 15 mg |
| Lactose | 57 mg |
| Microcrystalline cellulose | 25 mg |
| Magnesium stearate | 3 mg |
| | 120 mg |

Capsules The capsules containing 20 mg of the active ingredient are prepared according to the following formulation.

| | |
|---|---|
| Compound (I) | 20 mg |
| Corn starch | 30 mg |
| Lactose | 63 mg |
| Hydroxypropylcellulose | 6 mg |
| Magnesium stearate | 1 mg |
| | 120 mg |

While the present invention has been adequately and fully explained by the foregoing specification and examples included therein, it is subject to changes and modifications as long as they fall within the spirit and scope of the present invention.

**Claims**

1. A pyridine compound of the formula

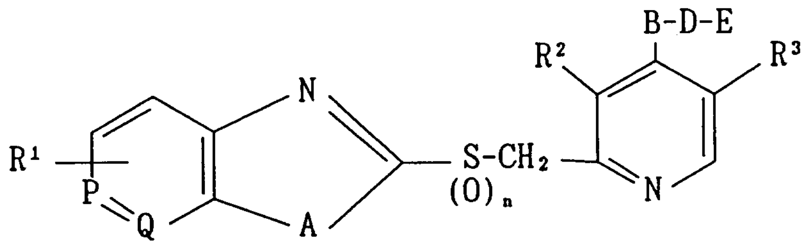

(I)

wherein

$R^1$ is a hydrogen, a halogen, an alkyl, an alkoxy, a hydroxyl, an alkoxycarbonyl, a carboxyl, a haloalkyl, a nitro, an amino, a mono- or dialkylamino, an alkoxycarbonylalkylamino or a carboxyalkylamino,

$R^2$ and $R^3$ are the same or different and each is a hydrogen, a halogen or an alkyl,

-P=Q- is -CH=CH-, -N=CH- or -CH=N-,

A is an oxygen atom, a sulfur atom or $N(R^4)$ wherein $R^4$ is hydrogen, alkyl, alkoxycarbonyl, hydroxyalkyl, alkoxyalkyl, acyloxyalkyl, alkoxycarbonylalkyl, carboxyalkyl, carbamoyl, carbamoylalkyl, mono- or dialkylcarbamoyl, mono- or dialkylcarbamoylalkyl, thiocarbamoyl, or mono- or dialkylthiocarbamoyl,

n is 0, 1 or 2,

B is an oxygen atom, S(O)p wherein p is 0, 1 or 2 or $N(R^5)$ wherein $R^5$ is hydrogen or alkyl,

D is a single bond, an alkylene, an alkylene having substituent or an alkylene having oxo, and

E is an alkoxyalkyl, a group of the formula (a)

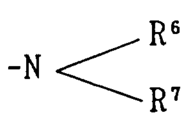

(a)

wherein $R^6$ and $R^7$ are the same or different and each is hydrogen, alkyl, cycloalkyl, acyl, alkoxycarbonyl, carbamoyl, mono- or dialkylcarbamoyl, optionally substituted phenylcarbamoyl, thiocarbamoyl, mono- or dialkylthiocarbamoyl, optionally substituted phenylthiocarbamoyl, hydroxyalkyl, alkoxycarbonylalkyl, optionally substituted phenylalkylcarbamoyl, optionally substituted phenylalkylthiocarbamoyl, carboxyalkyl, optionally substituted phenyl, optionally substituted phenylalkyl or optionally substituted heteroarylalkyl, or $R^6$ and $R^7$ may form, together with the adjoining nitrogen atom, an optionally condensed and optionally substituted heterocyclic ring, or a group of the formula (b)

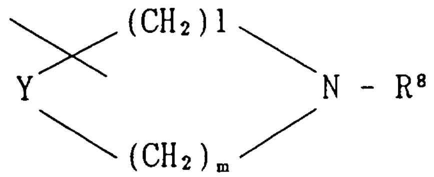

(b)

wherein $R^8$ is hydrogen, alkyl, acyl, carboxyalkyl or optionally substituted phenylalkyl, Y is methylene, oxygen atom or sulfur atom and l and m are the same or different and each is 0 or an integer of 1-3;

provided that when -P=Q- is -CH=CH- and A is $N(R^4)$, either one of the following applies:

(1) when $R^4$ is hydrogen, B is S(O)p wherein p is 0, 1 or 2 or $N(R^5)$ wherein $R^5$ is hydrogen or alkyl;

(2) when $R^4$ is carbamoyl or mono- or dialkylcarbamoyl, B is oxygen atom, D is alkylene and E is alkoxy, D is alkylene having 3 to 10 carbon atoms;

(3) when $R^4$ is carbamoyl or mono- or dialkylcarbamoyl, B is oxygen atom, D is alkylene and E is a group of the formula (a), D is alkylene having 5 to 10 carbon atoms or in the formula (a), $R^6$ and $R^7$ are the same or different and each is hydrogen, alkyl having 3 to 20 carbon atoms, cycloalky, acyl, optionally substituted phenyl, optionally substituted phenylalkyl or optionally substituted heteroarylalkyl, or $R^6$ and $R^7$ may form, together with the adjoining nitrogen atom, an optionally condensed and optionally substituted heterocyclic ring; and

(4) when $R^4$ is alkyl having 1 to 6 carbon atoms or alkoxycarbonyl having 2 to 7 carbon atoms, D is alkylene and E is alkoxy, B is S(O)p wherein p is 0, 1 or 2 or $N(R^5)$ wherein $R^5$ is hydrogen or alkyl;

or a pharmaceutically acceptable salt thereof.

**2.** The pyridine compound of Claim 1, having the formula (I) wherein B is S(O)p wherein p is 0, 1 or 2 and other symbols are as defined in Claim 1, or a pharmaceutically acceptable salt thereof.

**3.** The pyridine compound of Claim 1, which is selected from the group consisting of
2-(3-methyl-4-(2-morpholinoethylthio)-2-pyridyl)methylthio-1H-benzimidazole,
2-(3-methyl-4-(2-(1,2,3,4-tetrahydroisoquinolin-2-yl)ethylthio)2-pyridyl)methylthio-1H-benzimidazole,
2-(4-(2-(N-benzyl-N-methylamino)ethylthio)-3-methyl-2-pyridyl)methylthio-1H-benzimidazole,
2-(4-(1-benzyl-4-piperidyl)thio-3-methyl-2-pyridyl)methylthio-1H-benzimidazole,
2-((3-methyl-4-(2-(1,2,3,4-tetrahydroisoquinolin-2-yl)ethylthio)-2-pyridyl)methylthio)imidazo[5,4-b]-pyridine,
ethyl 2-(3-methyl-4-(2-morpholinoethylthio)-2-pyridyl)methylthio-1H-benzimidazole-1-carboxylate,
methyl 2-(3-methyl-4-(2-(1,2,3,4-tetrahydroisoquinolin-2-yl)ethylthio)-2-pyridyl)methylthio-1H-benzimidazole-1-carboxylate, 4-(2-((2-(1H-benzimidazol-2-yl)thiomethyl-3-methyl-4-pyridyl)thio)ethyl)-morpholine N-oxide,
2-(3-methyl-4-(2-acetylaminoethylthio)-2-pyridyl)methylthio-1H-benzimidazole,
2-(3-methyl-4-(2-benzylaminoethylthio)-2-pyridyl)methylthio-1H-benzimidazole,
2-(3-methyl-4-(2-(2-hydroxyethylamino)ethylthio)-2-pyridyl)methylthio-1H-benzimidazole,
2-(3-methyl-4-(2-(N,N-di(2-hydroxyethyl)amino)ethylthio)-2-pyridyl)methylthio-1H-benzimidazole,
2-(3-methyl-4-(2-(2-carboxyethylamino)ethylthio)-2-pyridyl)methylthio-1H-benzimidazole and
2-(3-methyl-4-(2-morpholino-2-carboxyethylthio)-2-pyridyl)methylthio-1H-benzimidazole, or a pharmaceutically acceptable salt thereof.

**4.** A pharmaceutical composition comprising the compound of Claim 1 and pharmaceutical additives.

**5.** An agent for preventing or treating various diseases caused by the bacteria belonging to the genus *Helicobacter*, which comprises the compound of Claim 1 as an active ingredient.

**6.** An agent for preventing or treating gastrointestinal diseases, which comprises the compound of Claim 1 as an active ingredient.

## INTERNATIONAL SEARCH REPORT

International application No. PCT/JP93/00732

**A. CLASSIFICATION OF SUBJECT MATTER**

Int. $Cl^5$ C07D401/12, 401/14, 413/12, 413/14, 417/12, 417/14, 471/04, 498/04, 513/04, A61K31/44, 31/535, 31/54, 31/55

According to International Patent Classification (IPC) or to both national classification and IPC

**B. FIELDS SEARCHED**

Minimum documentation searched (classification system followed by classification symbols)

Int. $Cl^5$ C07D401/12-401/14, 413/12-413/14, 417/12-417/14, 471/04, 498/04, 513/04

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X,Y | JP, A, 1-6270 (Eisai Co., Ltd.), January 10, 1989 (10. 01. 89), & EP, A, 268956 | 1-6 |
| X,Y | JP, A, 59-181277 (Aktiebolag Haessle), October 15, 1984 (15. 10. 84), & DE, A, 3404610 & FR, A, 2543551 | 1-6 |
| X,Y | JP, A, 58-39622 (Aktiebolag Haessle), March 8, 1983 (08. 03. 83), & EP, A1, 74341 & US, A, 4746667 | 1-6 |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| September 20, 1993 (20. 09. 93) | October 12, 1993 (12. 10. 93) |
| Name and mailing address of the ISA/ Japanese Patent Office | Authorized officer |
| Facsimile No. | Telephone No. |